# EUROPÄISCHE PATENTSCHRIFT

(11) **EP 0 362 575 B1**
(45) Veröffentlichungstag und Bekanntmachung des Hinweises auf die Patenterteilung: **12.04.1995**
(21) Anmeldenummer: 89116700.9
(22) Anmeldetag: 09.09.1989
(51) Int. Cl.: C07C 323/22, C07C 327/34, A61K 31/195, A61K 31/27

(54) **Neue organische Nitrate und Verfahren zu deren Herstellung**
Organic nitrates and method for their preparation
Nitrates organiques et procédé de leur préparation

(30) Priorität: 15.09.1988 DE 3831311
(43) Veröffentlichungstag der Anmeldung: 11.04.1990
(73) Patentinhaber: SCHWARZ PHARMA AG, D-40789 Monheim (DE)
(72) Erfinder: Hütter, Joachim, Dr.med., D-5090 Leverkusen 1 (DE); Sandrock, Klaus, Dr., D-4018 Langenfeld (DE); Noack, Eike, Prof. Dr.med., D-4040 Neuss 21 (DE)

(56) Entgegenhaltungen:
- EP-A- 0 200 915
- PATENT ABSTRACTS OF JAPAN, Band 7, Nr. 151 (C-174)(1296), 2. Juli 1983 & JP-A-5862147 (DAIICHI SEIYAKU K.K.) 13.04.1983
- CHEMICAL ABSTRACTS, Band 107, Nr. 13, 28. September 1987, Seite 43, Spalte 1, Zusammenfassung Nr. 109095p, Columbus, Ohio, US; M. FEELISH et al.: "Correlation between nitric oxide formation during degradation of organic nitrates and activation of guanylate cyclase" & Eur. J. Pharmacol. 1987, Bank 139, Nr. 1, Seiten 19-30
- CHEMICAL ABSTRACTS, Band 103, Nr. 25, 23. Dezember 1985, Seite 73, Spalte 2, Zusammenfassung Nr. 206021m, Columbus, Ohio, US; H. SCHROEDER et al.: "Evidence for a correlation between nitric oxide formation by cleavage of organic nitrates and activation of guanylate cyclase" & J. Mol. Cell. Cardiol. 1985, Bank 17, Nr. 9, Seiten 931-934
- CHEMICAL ABSTRACTS, Band 93, Nr. 19, 10. November 1980, Seite 260, Spalte 1, Zusammenfassung Nr. 181779z, Columbus, Ohio, US; L.J. IGNARRO et al.: "Requirement of thiols for activation of coronary arterial guanylate cyclase by glyceryl trinitrate and sodium nitrite. Possible involvement of s-nitrosothiols" & Biochim.Biophys. Acta 1980, Band 631, Nr. 2, Seite 221-231
- CHEMICAL ABSTRACTS, Band 92, Nr. 21, 26. Mai 1980, Seite 232, Spalte 2, Zusammenfassung Nr. 176369r, Columbus, Ohio, US; L.J. IGNARRO et al.: "Possible involvement of S-nitrosothiols in the activation of guanylate cyclase by nitroso compounds" & FEBS Lett. 1980, Band 110, Nr. 2, Seiten 275-278
- CHEMICAL ABSTRACTS, Band 109, Nr. 11, 12. September 1988, Seite 43, Spalte 1, Zusammenfassung Nr. 85991r, Columbus, Ohio, US; M. FEELISH et al.: "Explanation of the discrepancy between the degree of organic nitrate decomposition, nitrite formation and guanylate cyclase stimulation " & Eur. Heart J. 1988, Band 9 (Supp.A), Seiten 57-62

## Beschreibung

Die Erfindung betrifft neue organische Nitrate und Verfahren zu deren Herstellung.

Organische Nitrate (Salpetersäureester) haben sich in der Therapie von Herzerkrankungen bewährt.

Sie entfalten ihre Wirkung sowohl durch eine Herzentlastung über eine Senkung von Vor- und Nachlast als auch durch eine Verbesserung des Sauerstoffangebotes für das Herz über eine Erweiterung der Koronargefäße.

Allerdings hat man in den vergangenen Jahren festgestellt, daß die bisher in der Therapie eingesetzten organischen Nitrate, wie Glyceroltrinitrat (GTN), Isosorbid-5-mononitrat oder Isosorbiddinitrat bei hoher und kontinuierlicher Zufuhr zum Organismus innerhalb relativ kurzer Zeit eine deutliche Abschwächung der Wirkung, die Nitrattoleranz, aufweisen. Zahlreiche Experimente weisen darauf hin, daß die Anwesenheit von Sulfhydryl-Gruppen die Entstehung einer Nitrattoleranz verhindern und eine bereits eingetretene Toleranz abschwächen kann.

Der Mechanismus der Toleranzerzeugung wird heute so verstanden:
Nach derzeitigem Kenntnisstand ist die pharmakologische Wirkung der organischen Nitroverbindungen von der Anwesenheit von Cystein abhängig. Mit diesem bildet das organische Nitrat eine gemeinsame Vorstufe, aus deren Zerfall u.a. NO-Radikale freigesetzt werden, die das Zielenzym, die lösliche Guanylatzyklase der glatten Muskelzelle, aktiviert. Weitere, durch die Bildung von cGMP ausgelöste Folgereaktionen, führen dann zur Relaxation bzw. Gefäßdilatation.

Bei dem reaktiven und kurzlebigen, bisher noch hypothetischen Intermediärprodukt dürfte es sich um den Thioester der Salpetersäure oder ein Thionitrat handeln. Durch intramolekulare Umlagerung und weitere Folgereaktionen, die noch nicht aufgeklärt sind, wird letztlich die Bildung eines Nitrosothiols postuliert, aus dem dann Stickstoffmonoxid bzw. Nitritionen freigesetzt werden. Der enzymabhängige Abbau mit Hilfe der GSH-Reduktase dürfte dagegen, da er ausschließlich zur Bildung von Nitritionen führt, für die pharmakologische Wirkung ohne Bedeutung sein. Der nichtenzymatische Abbau benötigt also, wie geschildert, Cystein und ist damit dosisabhängig erschöpfbar (Erschöpfung des SH-Gruppen-pools), so daß auf Dauer nicht mehr genug NO als eigentlicher Aktivator der Guanylzyklase gebildet werden kann, und es klinisch zu einer Wirkungsabschwächung kommt.

Bei den erfindungsgemäßen Substanzen handelt es sich um spezifisch aufgebaute Verbindungen, die sich aus Nitratofettsäuren (Nitratoalkansäuren) und schwefelhaltigen Aminosäuren bzw. Peptiden zusammensetzen.

Aufgabe der vorliegenden Erfindung ist es daher, neue organische Nitrate zur Verfügung zu stellen, die sich aufgrund ihres allgemeinen Bauprinzipes - Anwesenheit von Sulfhydrylgruppen - dadurch auszeichnen, daß eine Nitrattoleranz oder eine bereits eingetretene Toleranz verhindert oder abgeschwächt wird.

Diese Aufgabe wurde dadurch gelöst, daß die Verbindungen die allgemeine Formel 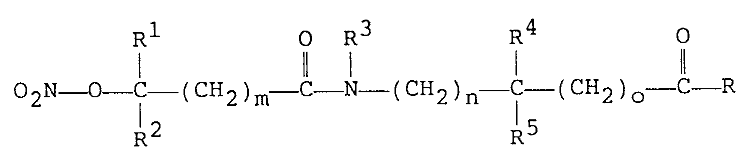
besitzen,
worin
- R: Hydroxy, C₁-C₃-alkoxy, Amino;
- R¹: Wasserstoff, Alkyl mit 1 bis 6 Kohlenstoffatomen;
- R²: Wasserstoff oder Methyl;
- R³: Wasserstoff;
- R⁴: Wasserstoff, Phenyl oder Methoxyphenyl;
- R⁵: Mercaptomethyl, Mercaptoethyl, Methylthioethyl, insbesondere S-acetyl, S-propionyl, S-butyryl, S-capronyl, S-capryl, S-isobutyryl, S-pivaloyl, S-benzoyl bedeuten,
- R und R⁵: miteinander unter Bildung eines Thiolactons, verbunden sein können,
- R und R⁴: miteinander unter Bildung eines Esters oder Amids verbunden sein können,
- R³ und R⁴: miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
m, n und o die Zahlenwerte 0 bis 10 bedeuten
und die pharmazeutisch annehmbaren Salze davon.
Nach einer Weiterbildung der Erfindung besitzen die Nitratofettsäurebestandteile eine Kettenlänge von C₂ - C₆; sie können geradkettig, verzweigt, racemisch oder optisch isomer sein.
Bevorzugt finden die Aminosäuren Cystein, Methionin oder Homocystein Verwendung.
Vorteilhafterweise weisen die Aminosäuren die stereochemische L-Form auf.
Cystein und/oder Methionin können in Form ihrer Methyl-, Ethyl- oder Propylester vorliegen.
Die SH-Gruppe des Cysteins kann mit Niederalkancarbonsäure mit 2 bis 8 Kohlenstoffatomen verestert sein.

Nach einer besonders vorteilhaften Weiterbildung der Erfindung weisen die beanspruchten Verbindungen die folgenden chemischen Formeln auf:
N-(2-Nitratoacetyl)-cysteinethylester
N-(2-Nitratoacetyl)-S-acetyl-cysteinethylester
N-(2-Nitratoacetyl)-S-propionyl-cysteinethylester
N-(2-Nitratoacetyl)-S-pivaloyl-cysteinethylester
N-(2-Nitratoacetyl)-methioninmethylester
N-(2-Nitratopropionyl)-cystein
N-(2-Nitratopropionyl)-cysteinethylester
N-(2-Nitratopropionyl)-methioninethylester
N-(2-Nitratobutyryl)-cystein
N-(2-Nitratobutyryl)-cysteinethylester
N-(2-Nitratobutyryl)-S-acetyl-cysteinethylester
N-(2-Nitratobutyryl)-S-butyryl-cysteinethylester
N-(2-Nitratobutyryl)-methioninethylester
N-(2-Nitratoisobutyryl)-cystein
N-(2-Nitratoisobutyryl)-cysteinethylester
N-(2-Nitratoisobutyryl)-S-benzoyl-cysteinethylester
N-(2-Nitratoisobutyryl)-S-acetyl-cysteinethylester
N-(2-Nitratoisobutyryl)-S-pivaloyl-cysteinethylester
N-(2-Nitratoisobutyryl)-methioninethylester
N-(3-Nitratobutyryl)-cystein
N-(3-Nitratobutyryl)-cysteinethylester
N-(3-Nitratobutyryl)-S-acetyl-cysteinethylester
N-(3-Nitratobutyryl)-S-propionyl-cysteinethylester
N-(3-Nitratobutyryl)-methioninethylester
N-(3-Nitratobutyryl)-homocysteinthiolacton
N-(3-Nitratopivaloyl)-cystein
N-(3-Nitratopivaloyl)-cysteinethylester
N-(3-Nitratopivaloyl)-cysteinethylester-S-carbonat
N-(3-Nitratopivaloyl)-S-acetyl-cysteinethylester
N-(3-Nitratopivaloyl)-S-propionyl-cysteinethylester
N-(3-Nitratopivaloyl)-S-butyryl-cysteinethylester
N-(3-Nitratopivaloyl)-S-isobutyryl-cysteinethylester
N-(3-Nitratopivaloyl)-S-pivaloyl-cysteinethylester
N-(3-Nitratopivaloyl)-S-benzoyl-cysteinethylester
N-(3-Nitratopivaloyl)-methioninethylester
N-(3-Nitratopivaloyl)-methionin
N-(3-Nitratopivaloyl)-homocysteinthiolacton
N-(2-Nitratohexanoyl)-cysteinethylester
N-(2-Nitratohexanoyl)-S-propionyl-cysteinethylester
N-(3-Nitratohexanoyl)-cysteinethylester
N-(3-Nitratohexanoyl)-methioninmethylester
N-(12-Nitratolauroyl)-cystein
N-(12-Nitratolauroyl)-cysteinethylester
N-(12-Nitratolauroyl)-S-acetyl-cystein
N-(12-Nitratolauroyl)-S-pivaloyl-cystein
Nach einer anderen Ausbildung der Erfindung weisen Arzneimittel einen Gehalt an einer und/oder einem Gemisch der erfindungsgemäßen Verbindungen auf.

Diese Arzneimittel können zur Behandlung von Kreislauferkrankungen, beispielsweise als Coronardilatatoren, als Mittel zur Behandlung von Hochdruck, Herzinsuffizienz und zur Erweiterung der peripheren Gefäße, einschließlich der Hirn- und Nierengefäße eingesetzt werden.

Schließlich können die Verbindungen in an sich bekannter Weise hergestellt werden, indem die entsprechenden Nitratofettsäuren oder deren reaktionsfähige Derivate mit der Aminogruppe einer Aminosäure bzw. eines Peptids kondensiert werden. Die erhaltenen Verbindungen können ggfs. in einem weiteren Reaktionsschritt einer Seitenkettenalkylierung oder - acylierung unterworfen werden.

Reaktionsfähige Derivate der erfindungsgemäß eingesetzten Nitratofettsäuren sind beispielsweise Säurehalogenide, Säureanhydride, aktivierte Amide oder aktivierte Ester. Bevorzugt werden Säurechloride, Säureazide, symmetrische Säureanhydride, aktivierte Ester und gemischte Anhydride mit organischen oder anorganischen Säuren.

Die Kondensationsreaktionen von Nitratofettsäuren mit Aminogruppen von Aminosäuren können auch in einem inerten Lösungsmittel und in Gegenwart eines die Bildung von Säureamidbindungen fördernden Kondensationsmittels, eines Carbodiimids, wie N,N'-Dicyclohexylcarbodiimid oder eines ähnlichen Carbodiimids, einer Iminverbindung, wie Diphenylketen-N-cyclohexylimin oder Pentamethylen-keten-N-cyclohexylimin oder eines Phosphats oder Phosphits, wie Triethylphosphit, Ethylpolyphosphat oder Isopropylpolyphosphat während eines Zeitraums von 1 - 48 Std. bei Temperaturen von -10° C bis zur Rückflußtemperatur des verwendeten Lösungsmittels durchgeführt werden.

Die Ausführungsbeispiele, ohne darauf beschränkt zu sein, erläutern die Erfindung.

### Beispiel 1

### Darstellung von N-(3-Nitratobutyryl)-cysteinethylester

### 1. Arbeitsschritt Verseifung des 3-Hydroxybuttersäureethylesters

13,2 g (0,1 mol) 3-Hydroxybuttersäureethylester (Aldrich) wurden mit 4,0 g (0,1 mol) in 100 ml Wasser gelöstem NaOH versetzt. Die Reaktion war beendet, als die Lösung homogen geworden war.

Die Aufarbeitung erfolgte, indem die erhaltene Lösung mit 10 ml HCl konz. angesäuert wurde und zweimal mit je 100 ml Ethylacetat extrahiert wurde. Anschließend wurde die Lösung einrotiert, wobei ein dünnflüssiges Öl zurückblieb.

Die Ausbeute betrug 8,81 g (theoretisch: 10,4 g) 3-Hydroxybuttersäure.

### 2. Arbeitsschritt Nitrierung der 3-Hydroxybuttersäure

8,81 g (0,08 mol) 3-Hydroxybuttersäure und 50 mg Harnstoff wurden in 50 ml Essigsäure bei 5° C gelöst. Zunächst wurden 6,27 ml (0,15 mol) HNO₃, anschließend unter Kühlung 14,17 ml (0,15 mol) Ac₂O zugetropft. Das Reaktionsgemisch wurde über Nacht gerührt.

Die Aufarbeitung erfolgte, indem die erhaltene Lösung mit 200 ml Eiswasser versetzt und mit Ethylacetat extrahiert wurde. Die organische Phase wurde mit NaHCO₃ extrahiert. Die NaHCO₃-Phase wurde mit HCl-konz. angesäuert und mit Ethylacetat extrahiert. Schließlich wurde die Lösung einrotiert, wobei ein dünnflüssiges Öl zurückblieb.

Die Ausbeute betrug 9,4 g (theoretisch 11,9 g) 3-Nitratobuttersäure.

### 3. Arbeitsschritt Darstellung von N-(3-Nitratobutyryl)-cysteinethylester

16,6 g (0,11 mol) 3-Nitratobuttersäure werden in 100 ml Dichlormethan gelöst. Unter N₂-Zufuhr, bei 15° C, wird langsam 17,9 g (0,12 mol) cysteinethylester zugegeben. Anschließend läßt man langsam, bei 15° C und N₂-Zufuhr, 24,7 g (0,12 mol) Dicyclohexylcarbodiimid (DCC), gelöst in 80 ml Dichlormethan, zutropfen. Nach Reaktionsende wird der entstandene Dicyclohexyl-Harnstoff abgesaugt und die Lösung mit 150 ml 0,1 N HCl gewaschen. Danach wird die Lösung einrotiert.

Die Aufreinigung der Substanz erfolgte durch präparative Säulenchromatografie und Umkristallisation aus Ethanol/n-Hexan.
Ausbeute: 6,88 g (theoretisch: 30,83 g)
Fp: 77,8° C.

### Beispiel 2

### Darstellung von N-(3-Nitratobutyryl)-methioninethylester

6,35 g (0,043 mol) 3-Nitratobuttersäure, 7,47 g (0,043 mol) Methioninethylester und eine Spatelspitze Dimethylaminopyridin (DMAP) wurden unter Rühren und Kühlen auf 10° C in 100 ml Dichlormethan gelöst. 10,31 g (0,05 mol) DCC wurden in 80 ml CH₂Cl₂ gelöst und unter gleichzeitiger Zufuhr von Stickstoff langsam zugetropft. Nach Reaktionsende wurde die Lösung abgesaugt, mit NaHCO₃ und schließlich mit HCl gewaschen. Die Lösung wurde einrotiert, wobei ein Öl zurückblieb.

Die Aufreinigung erfolgte durch präparative Säulenchromatografie oder durch Kristallisation in der Kälte.

Die Ausbeute betrug 1,95 g (theoretisch 12,05 g) N-(3-Nitratobutyryl)-methioninethylester als farbloses Öl.

### Beispiel 3

### Darstellung von N-(3-Nitratopivaloyl)-cysteinethylester

### 1. Arbeitsschritt Darstellung von Nitratopivalinsäuremethylester

25,0 g (0,19 mol) Hydroxypivalinsäuremethylester und 0,12 g Harnstoff wurden bei Raumtemperatur in 250 ml CH₂Cl₂ gelöst und unter Rühren auf 5° C abgekühlt. Hierzu wurde unter Rühren 23,8 g (0,38 mol) HNO₃ (100 %ig) so zugetropft, daß die Temperatur 10° C nicht überschritt. Anschließend wurde auf 5° C abgekühlt und unter Rühren 38,6 g (0,38 mol) Essigsäureanhydrid in der Weise zugetropft, daß die Temperatur 10 ° C nicht überschritt. 15 Min. wurde unter Kühlung im Eisbad gerührt, anschließend langsam auf Raumtemperatur erwärmt und über Nacht bei RT weitergerührt. Der Ansatz wurde unter Rühren langsam auf 500 ml Eiswasser gegeben. Die CH₂Cl₂-Phase wurde abgetrennt und je einmal mit 100 ml H₂O dest., 100 ml gesättigter, wässriger NaHCO₃-Lösung und nochmals 100 ml H₂0 dest. gewaschen. Der CH₂Cl₂-Extrakt wurde dann am Rotavapor bei einer Bad-Temperatur von max. 40° C im Wasserstrahlvakuum bis zur Trockne eingeengt. Der hellgelbe, ölige Rückstand destillierte im Ölpumpenvakuum bei 60° C Badtemperatur als klares, dünnflüssiges Öl über.
Ausbeute: 31,5 g ≙ 94,0 % der Theorie

### 2. Arbeitsschritt Darstellung von Nitratopivalinsäure

14,0 g (0,350 mol) NaOH wurden in H₂0 dest. gelöst und auf ca. 10°C abgekühlt. Hierzu wurde unter Rühren eine Lösung von 31,0 g (0,175 mol) Nitratopivalinsäuremethylester in 250 ml Methanol zugegeben, wobei sich das Reaktionsgemisch gelblich verfärbte und die Temperatur auf ca. 25° C steigerte. Der Ansatz wurde nach 90 Min. Rühren mit 29,5 ml (0,35 mol) 37 %iger HCl neutralisiert und das Methanol am Rotavapor vollständig abdestilliert. Die wässrige Phase wurde zweimal mit je 200 ml Methylenchlorid extrahiert. Die vereinigten Methylenchloridextrakte wurden einmal mit 50 ml H₂O dest. gewaschen und die Methylenchlorid-Phase am Rotavapor bis zur Trockne eingeengt. Der farblose, ölige Rückstand wurde in 100 ml Ethylacetat gelöst und erneut am Rotavapor bis zur Trockne eingeengt, wobei ein fester, weißer Rückstand zurückblieb, aus dem Lösungsmittelreste am Ölpumpenvakuum (0,4 Torr) bei einer Bad-Temperatur von ca. 40° C in 15 Min. am Rotavapor entfernt wurden. Der feste, weiße Rückstand von 25,44 g (89,1 % der Theorie) wurde in 100 ml siedendem n-Hexan gelöst und mit 2 ml Diisopropylether versetzt. Nach Abkühlen auf Raumtemperatur und Zugabe von Impfkristallen kristallisierte das Produkt aus. Das Produkt wurde 72 Stunden bei 0° C belassen, die Kristalle abgesaugt und nach Waschen mit zweimal je 10 ml n-Hexan im Vakuum-Trockenschrank bis zur Gewichtskonstanz bei Raumtemperatur bei ca. 2 Torr getrocknet.
Fp: 54,2° C.
Ausbeute: 23,66 g ≙ 82,9 % der Theorie

### 3. Arbeitsschritt Darstellung von N-(3-Nitratopivaloyl)-cysteinethylester

10,7 g (71,7 mmol) L-cysteinethylester Base wurde unter N₂₋ Atmosphäre in 200 ml Methylenchlorid bei Raumtemperatur unter Rühren gelöst. Hierzu wurden 11,4 g (70,0 mmol) kristalline Nitroxypivalinsäure zugegeben und unter Rühren bei Raumtemperatur gelöst. Zu dieser Mischung wurde unter Rühren und unter Stickstoff-Atmosphäre eine Lösung von 14,8 g (71,7 mmol) N-N-Dicyclohexylharnstoff (DCC) in 50 ml Methylenchlorid bei Raumtemperatur in ca. 15 Min. zugetropft, wobei die Temperatur auf 35° C anstieg. Nach weiterem Rühren fiel weißer Dicyclohexylharnstoff aus. Dar Ansatz wurde auf Raumtemperatur abgekühlt und über Nacht unter Stickstoff-Atmosphäre gerührt. Der Dicyclohexylharnstoff wurde danach über eine Glasfilterfritte abgesaugt und einmal mit 50 ml CH₂Cl₂ gewaschen. Die vereinigten Methylenchlorid-Lösungen wurden einmal mit 100 ml 1 n HCl und zweimal mit je 100 ml H₂O dest. gewaschen (unter N₂-Atmosphäre) und dann im Rotavapor bei einer Bad-Temperatur von ca. 40° C und Wasserstrahlvakuum von anfangs 550 mbar bis ca. 20 mbar eingeengt. Es wurde ein hellbraunes Öl erhalten.
Ausbeute: 21,2 g ≙ 102,9 % der Theorie
Die Substanz wurde durch Kristallisation aus Ethanol/Hexan in der Kälte gereinigt.
Ausbeute: 13,42 g ≙ 65,1 % der Theorie N-(3-Nitratopivaloyl)-cysteinethylester als hellrosa Öl.

### 4. Arbeitsschritt Darstellung von N-(3-Nitratopivaloyl)-S-acetyl-cysteinethylester

Zu 10,3 g (35,0 mmol) N-(3-Nitratopivaloyl)-cysteinethylester gelöst in 70 ml Dichlormethan wurde in der Kälte eine Lösung von 4,3 g (42,0 mmol) Essigsäureanhydrid in 10 ml Dichlormethan unter Rühren zugetropft. Anschließend wurde eine Lösung von 5,0 g (49,0 mmol) Triethylamin in 20 ml Dichlormethan in der Kälte unter Rühren zugetropft. Nach Reaktionsende wurde der Ansatz mit 1 n HCl, 10 %iger wässriger Natriumbicarbonat-Lösung und Wasser gewaschen. Der Dichlormethan-Extrakt wurde am Rotavapor bis zur Trockne eingeengt. Es wurden 11,6 g hellgelbes, öliges Produkt erhalten, aus welchem durch Kristallisation aus Ethanol/Wasser in der Kälte durch Zugabe von Impfkristallen 7,8 g kristallines Produkt (≙ 66,3 % der Theorie) erhalten wurden.
Fp: < 5° C.

### 4. Arbeitsschritt/Variante 1 Darstellung von N-3(Nitratopivaloyl)-S-butyryl-cysteinethylester

Setzt man 6,7 g (42,0 mmol) Buttersäureanhydrid anstelle des im Arbeitsschritt 4 beschriebenen Essigsäureanhydrid ein, erhält man bei gleicher Reaktionsführung und Aufarbeitung 13,0 g hellgelbes, öliges Produkt, aus welchem, wie im Arbeitsschritt 4 beschrieben, durch Kristallisation in der Kälte 9,7 g kristallines Produkt (≙ 76,2 % der Theorie) erhalten wurden.
Fp: < 5° C.

### 4. Arbeitsschritt/Variante 2 Darstellung von N-(3-Nitratopivaloyl)-S-pivaloyl-cysteinethylester

Setzt man 7,8 g (42,0 mmol) Pivalinsäureanhydrid anstelle des im Arbeitsschritt 4 beschriebenen Essigsäureanhydrid ein, so erhält man bei gleicher Reaktionsführung und Aufarbeitung 14,1 g hellgelbes, öliges Produkt, aus welchem man, wie im Arbeitsschritt 4 beschrieben, durch Kristallisation 10,5 g kristallines Produkt (≙ 79,5 % der Theorie) erhält.
Fp: 45° C.

### 4. Arbeitsschritt/Variante 3 Darstellung von N-(3-Nitratopivaloyl)-cysteinethylester-S-carbonat

Setzt man 4,3 g (42,0 mmol) Chlorameisensäureethylester anstelle des im Arbeitsschritt 4 beschriebenen Essigsäureanhydrid ein, so erhält man bei gleicher Reaktionsführung und Aufarbeitung 11,5 g hellgelbes öliges Produkt, aus welchem man, wie im Arbeitsschritt 4 beschrieben, durch Kristallisation 9,5 g kristallines Produkt (≙ 74,1 % der Theorie) erhält.
FP: 36° C.

### Beispiel 4

### Darstellung von N-(3-Nitratopivaloyl)-methioninethylester

12,4 g (70,0 mmol) L-Methioninethylester Base wurden unter N₂-Atmosphäre in 250 ml Methylenchlorid bei Raumtemperatur unter Rühren gelöst. Hierzu wurden 11,4 g (70,0 mmol) kristalline Nitratopivalinsäure zugegeben und unter Rühren bei Raumtemperatur gelöst. Zu dieser Mischung wurde unter Rühren und unter Stickstoff-Atmosphäre eine Lösung von 14,8 g (71,7 mmol) N-N-Dicyclohexylharnstoff (DCC) in 50 ml Methylenchlorid bei Raumtemperatur in ca. 15 Min. zugetropft, wobei die Temperatur auf 35° C anstieg. Nach weiterem Rühren fiel weißer Dicyclohexyl-Harnstoff aus. Der Ansatz wurde auf Raumtemperatur abgekühlt und über Nacht unter Stickstoff-Atmosphäre gerührt. Der DCC-Harnstoff wurde danach über eine Glasfilterfritte abgesaugt und einmal mit 50 ml CH₂Cl₂ gewaschen. Die vereinigten Methylenchloridlösungen wurden einmal mit 100 ml 1 n HCl und zweimal mit je 100 ml H₂O dest. gewaschen (unter N₂-Atmosphäre) und dann im Rotavapor bei einer Bad-Temperatur von ca. 40° C und Wasserstrahlvakuum von anfangs 550 mbar bis ca. 20 mbar eingeengt. Es wurde hellgelbes Öl erhalten.
Ausbeute: 24,9 g ≙ 110,3 % der Theorie roher N-(3-Nitratopivaloyl-L-methioninethylester.

Das Rohprodukt wurde mittels präparativer Säulenchromatographie gereinigt.
Ausbeute: 17,6 g ≙ 78,0 % der Theorie N-(3-Nitratopivaloyl)-methioninethylester als farbloses Öl.

### Beispiel 5

### N-(12-Nitratolauroyl)-S-acetyl-cystein

### 1. Arbeitsschritt Darstellung von 12-Nitratolaurinsäure

54,1 g (0,250 mol) 12-Hydroxylaurinsäure und 0,3 g Harnstoff wurden unter leichtem Erwärmen in 1,3 l CHCl₃ gelöst und unter Rühren auf 20° C gekühlt. Unter Rühren wurde langsam 23,6 g (0,375 mol) HNO₃ (100 %ig) zugetropft, wobei die Temperatur auf 27° C anstieg. Anschließend wurde auf 20° C abgekühlt und unter Rühren 38,3 g (0,375 mol) Essigsäureanhydrid unter Kühlen zugetropft, wobei ein Temperaturlimit von 25° C eingehalten wurde. Bei Raumtemperatur wurde über Nacht gerührt. Schließlich wurde fünfmal mit je 0,5 l H₂O dest. gewaschen. Die über Na₂SO₄ getrocknete und mit pulverisierter Aktivkohle geklärte CHCl₃-Phase wurde am Rotavapor bei einer Bad-Temperatur von 50° C unter Wasserstrahlvakuum bis zur Trockne eingeengt. Der ölige Rückstand von 60,8 g wurde in 500 ml siedendem n-Hexan gelöst und nach Abkühlen auf Raumtemperatur über Nacht im Kühlschrank bei 0° C belassen. Das auskristalliisierte Produkt wurde abgesaugt und zweimal mit je 50 ml n-Hexan gewaschen. Schließlich wurde das Produkt im Vakuum-Trockenschrank bei Raumtemperatur und ca. 2 Torr bis zur Gewichtskonstanz getrocknet.
Fp: 29° C.
Ausbeute: 39,4 g ≙ 60,3 % der Theorie

### 2. Arbeitsschritt Darstellung von 12-Nitratolaurinsäurechlorid

2,61 g (10 mmol) Nitratolaurinsäure wurden in 50 ml Methylenchlorid gelöst und 4,44 g (35 mmol) Oxalylchlorid in 50 ml Methylenchlorid bei Raumtemperatur unter Rühren zugetropft. Es wurde über Nacht gerührt. Schließlich wurde das Produkt am Rotationsverdampfer zur Trockne eingeengt.
Ausbeute: 3 g ≙ 93,2 % der Theorie

### 3. Arbeitsschritt Darstellung von N-(12-Nitratolauroyl)-cystein

Unter Stickstoffatmosphäre wurden 6,06 g (50 mmol) L-cystein in 300 ml DMF unter Rühren eingetragen. 5,60 g (20 mmol) 12-Nitratolaurinsäurechlorid wurden in 50 ml Dichlormethan zugetropft. Da keine klare Lösung erhalten wurde, wurde auf 60° C erwärmt. Schließlich wurden 100 ml H₂O dest. zugegeben und über Nacht bei Raumtemperatur gerührt. Anschließend wurde mit 300 ml H₂O dest. verdünnt und viermal mit je 200 ml Ethylacetat extrahiert. Die organische Phase wurde über Na₂SO₄ getrocknet und anschließend eingeengt. Der Rückstand wurde in 100 ml Äther aufgenommen und zum Auskristallisieren über Nacht im Kühlschrank bei 0° C belassen. Erhalten wurden weiße Kristalle.
Fp: 74 - 75° C.
Ausbeute: 4,1 g N-(12-Nitratolauroyl)-cystein.

### 4. Arbeitsschritt Darstellung von N-(12-Nitratolauroyl)-S-acetyl-cystein

Unter Stickstoff-Atmosphäre wurden 1,82 g (5 mmol) N-(12-Nitratolauroyl)-cystein in 20 ml Ethylacetat vorgelegt. Anschließend wurde auf 0° C abgekühlt und 2,5 ml Essigsäureanhydrid zugetropft. Dann wurden bei -5° C 1,52 g (15 mmol) Triethylamin, das in 5 ml Ethylacetat gelöst war, langsam zugetropft. Die Reaktionslösung wurde mit Wasser gewaschen und zur Trockne eingeengt.
Fp: bei RT Öl.
Ausbeute: 2 g ≙ 98,4 % der Theorie

### Beispiel 6

### Darstellung von N-(12-Nitratolauroyl)-cysteinethylester

4 g (26,8 mmol) Cysteinethylester-Base wurden in 50 ml Methylenchlorid gelöst und unter Rühren 2,8 g (10 mmol) 12-Nitratolaurinsäurechlorid, das in 50 ml Methylenchlorid gelöst war, zugetropft und über Nacht gerührt. Der ausgefallene cysteinethylester · HCl wurde abgesaugt und das Lösungsmittel am Rotavapor abgezogen. Der ölige Rückstand (6 g) wurde in 100 ml Äther gelöst und über Nacht bei 0° C im Kühlschrank belassen. Das ausgefallene Produkt wurde abgesaugt.
Fp: 59 - 60° C.
Ausbeute: 1,6 g ≙ 40,0 % der Theorie

### Beispiel 7

### Herstellung von N-(2-Nitratopropionyl)-cysteinethylester

### 1. Arbeitsschritt Darstellung von Nitratomilchsäureethylester

33 g (0,28 mol) Milchsäureethylester werden in 300 ml Dichlormethan gelöst. Nach Zugabe von 100 mg Harnstoff werden bei einer Temperatur von 5 - 10° C 22,5 ml (0,56 mol) 100 %ige Salpetersäure zugetropft. Die Lösung wird auf 0° C gekühlt. Dann werden 52,8 ml (0,56 mol) Acetanhydrid so zugetropft, daß die Temperatur 5° C nicht übersteigt. Die Lösung wird über Nacht bei Raumtemperatur stehen gelassen und dann mit 250 ml Wasser gewaschen. Die organische Phase wird abgetrennt und über Natriumsulfat getrocknet. Nach Filtration wird das Dichlormethan abdestilliert. Der erhaltene, ölige Rückstand wird destillativ aufgearbeitet.
Ausbeute: 30,34 g ≙ 66,4 % der Theorie
Kp: 34° C (0,25 Torr)

### 2. Arbeitsschritt Darstellung von Nitratomilchsäure

30 g (0,18 mol) Nitratomilchsäureethylester werden in 80 ml Dioxan gelöst. Die Lösung wird mit 30 ml Wasser und 2 g (0,02 mol) Schwefelsäure versetzt und 19 h refluxiert. Die Lösung wird auf ein Volumen von ca. 50 ml eingeengt und dann mit 300 ml Wasser verdünnt. Der pH-Wert wird durch Zugabe von Natriumhydrogencarbonat auf 7 - 8 eingestellt. Der nicht umgesetzte Ester wird durch Extraktion mit Dichlormethan entfernt.

Die wässrige Phase wird mit konz. Salzsäure auf pH 1 gestellt und dreimal mit je 150 ml Ethylacetat extrahiert. Die Extrakte werden vereinigt und über Natriumsulfat getrocknet. Nach Filtration wird das Ethylacetat vollständig am Rotationsverdampfer abgezogen.
Ausbeute: 14,6 g farbloses Öl ≙ 59,2 % der Theorie.

### 3. Arbeitsschritt Darstellung von N-(2-Nitratopropionyl)-cysteinethylester

Unter Stickstoffatmosphäre werden 17 g (0,13 mol/Nitratomilchsäure und 18,9 g (0,13 mol) cysteinethylester bei 10 - 15° C in 200 ml Dichlormethan gelöst. Bei 15-20° C wird eine Lösung von 28,6 (0,14 mol) N-N-Dicyclohexylcarbodiimid und 75 ml Dichlormethan zugetropft. Nach 1 h wird der ausgefallene N-N-Dicyclohexylharnstoff abfiltriert und mit 75 ml Dichlormethan nachgewaschen. Das Filtrat wird zweimal mit je 50 ml 0,1 n Salzsäure extrahiert. Die organische Phase wird am Rotationsverdampfer vollständig eingeengt. Das kristalline Rohprodukt (22,4 g) wird aus 100 ml Ethanol/n-Hexan (1:1) umkristallisiert.
Ausbeute: 7,6 g ≙ 22,6 % der Theorie.
Fp: 92,8° C

Die pharmakologische Wirkung der erfindungsgemäßen Verbindungen wird durch die Versuchsanordnungen weiter erläutert.

### Pharmakologische Versuchsanordnung 1

Einfluß neuer organischer Nitrate auf Kreislaufparameter am wachen Hund zum Nachweis der Nitratwirkung.

Prüfziel ist festzustellen, wie die neuen organischen Nitrate nach intravenöser und oraler Applikation auf verschiedene Kreislaufparameter am wachen Hund wirken. Alle Versuche wurden an trainierten Beagle-Hunden durchgeführt; die Kreislaufparameter wurden mit einem arteriellen Katheter-Tipmanometer und einem über die V. Jugularis eingeführten Einschwemmkatheter gemessen. Zur Beschreibung der Wirkung am arteriellen System wurden systolischer (SAP systolic arterial pressure), mittlerer (MAP mean arterial pressure) und diastolischer (DAP diastolic arterial pressure) Blutdruck (BP) und Herzfreqeunz (HR heart rate) gemessen. Daraus wurden der periphere Widerstand (TPR total periphere resistance) und die Dehnbarkeit des arteriellen Windkessels (COMPL) berechnet. Das Niederdrucksystem wurde durch den zentralvenösen Druck (CVP central venous pressure) und den pulmonalarteriellen Druck (PAP pulmonal arterial pressure) beschrieben. Als Referenzsubstanz wurde Isosorbid-5-mononitrat (ISM-5) verwendet.

Die folgenden Abbildungen 1 und 2 erläutern in graphischer Darstellungsweise das Wirkungsspektrum der erfindungsgemäßen organischen Nitrate.

In Abbildung 1 sind die Wirkungen von oral und intravenös verabreichtem ISM-5 dargestellt. Nach beiden Applikationen senkt ISM-5 den systolischen Blutdruck leicht, der Mitteldruck wird kaum beeinflußt, die Dehnbarkeit des Windkessels steigt deutlich an und die Drucke im Niederdrucksystem nehmen ab.

Abbildung 2 zeigt, die entsprechenden Wirkungen von N-(3-Nitratopivaloyl)-methioninethylester (Nitrato-Piv-Meth-Et) in den entsprechenden Kreislaufabschnitten. Auch hier weist der Vergleich zwischen intravenöser und oraler Applikation auf eine gute Bioverfügbarkeit hin.

Die Substanz N-(3-Nitratopivaloyl)-cysteinethylester zeigt ebenfalls eine gute Bioverfügbarkeit und einen nitrattypischen Wirkungsverlauf.

Die Ergebnisse zeigen, daß beide geprüften Substanzen eine mit ISM-5 vergleichbare Wirkung mit guter Bioverfügbarkeit besitzen.

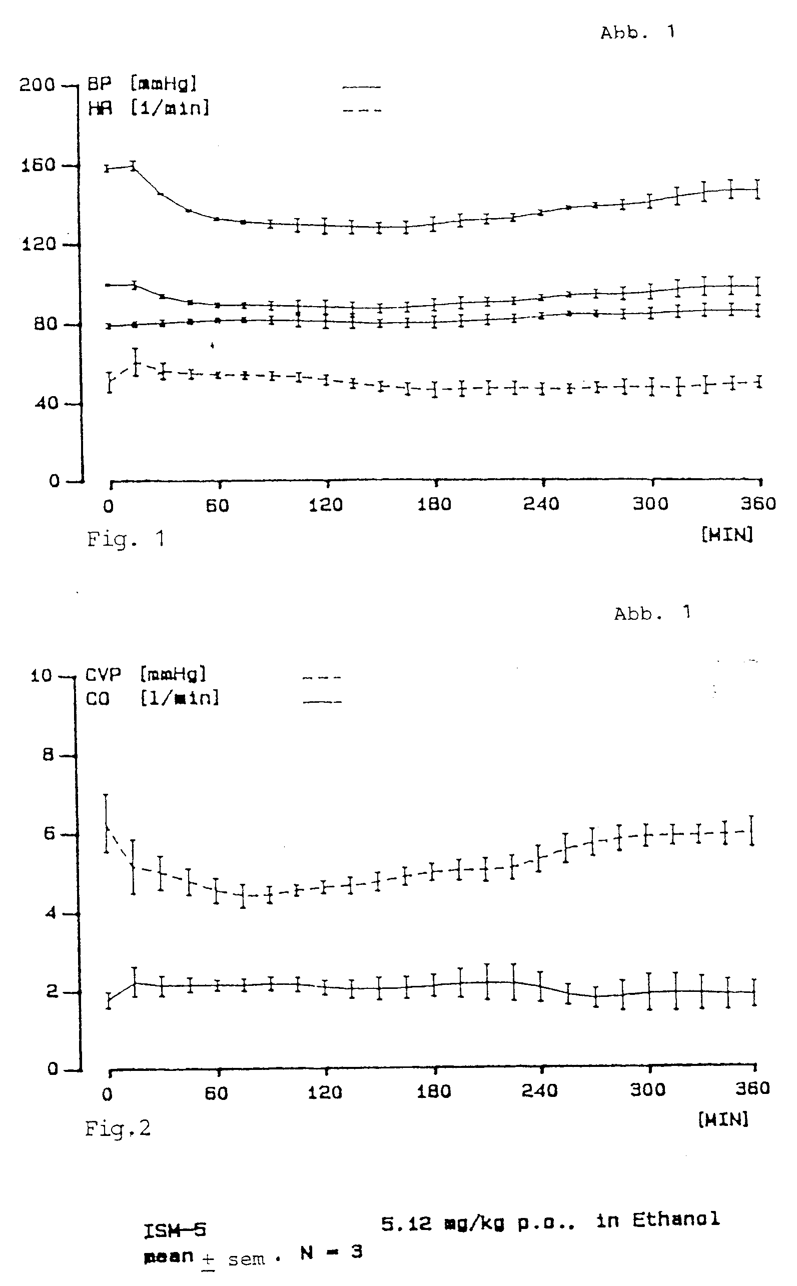

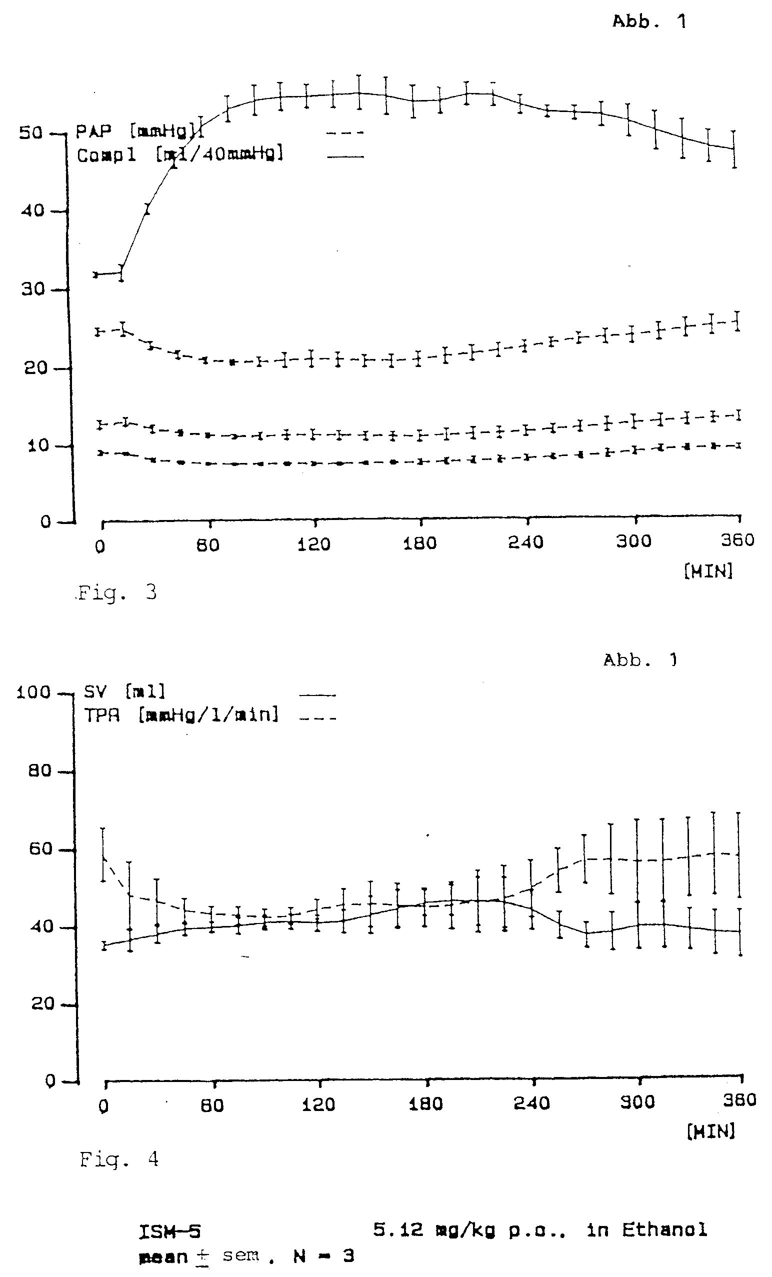

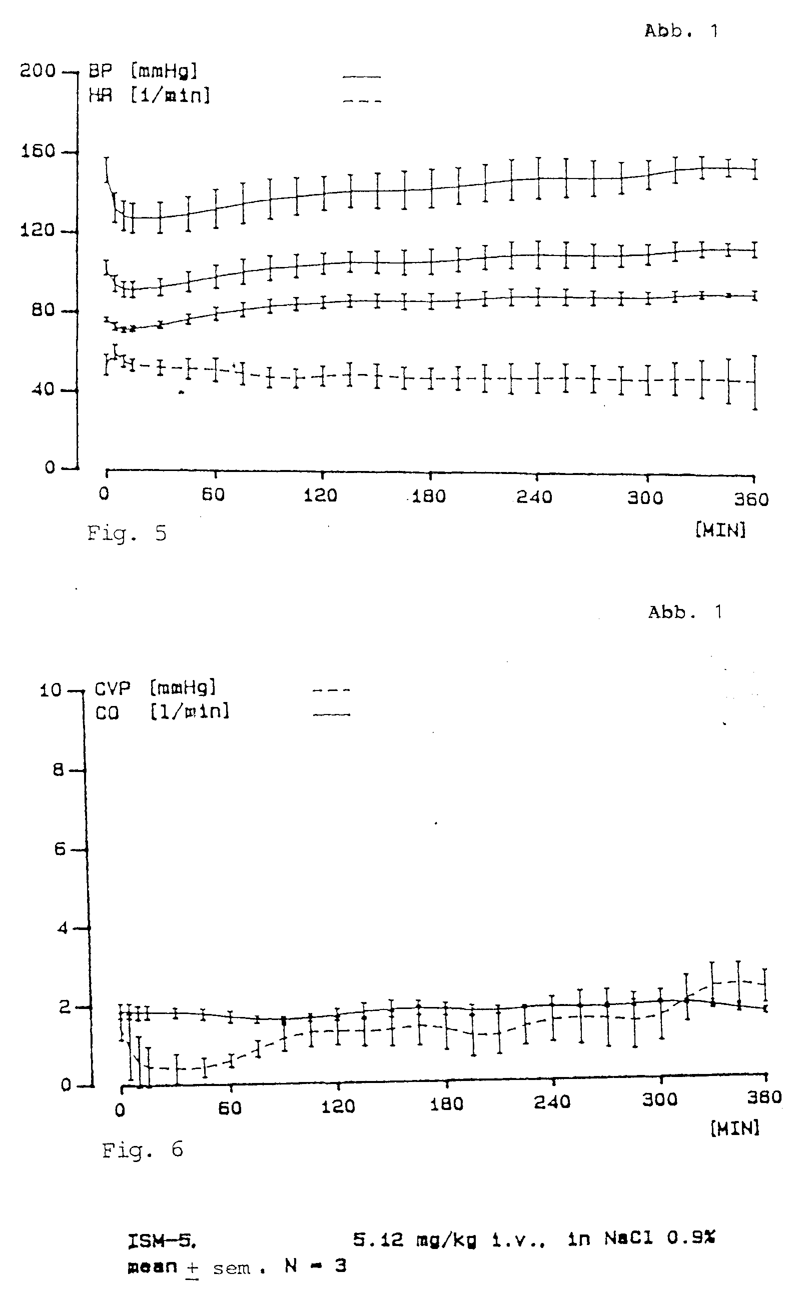

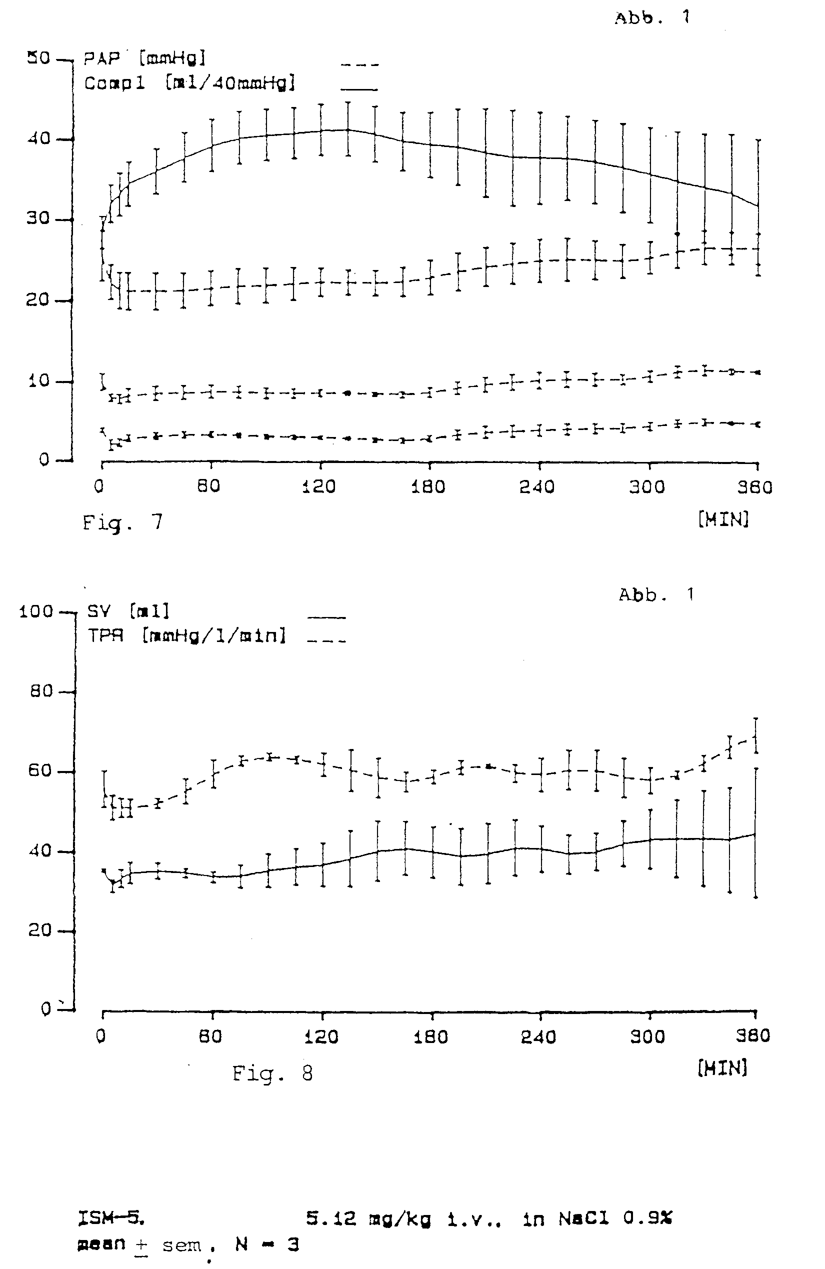

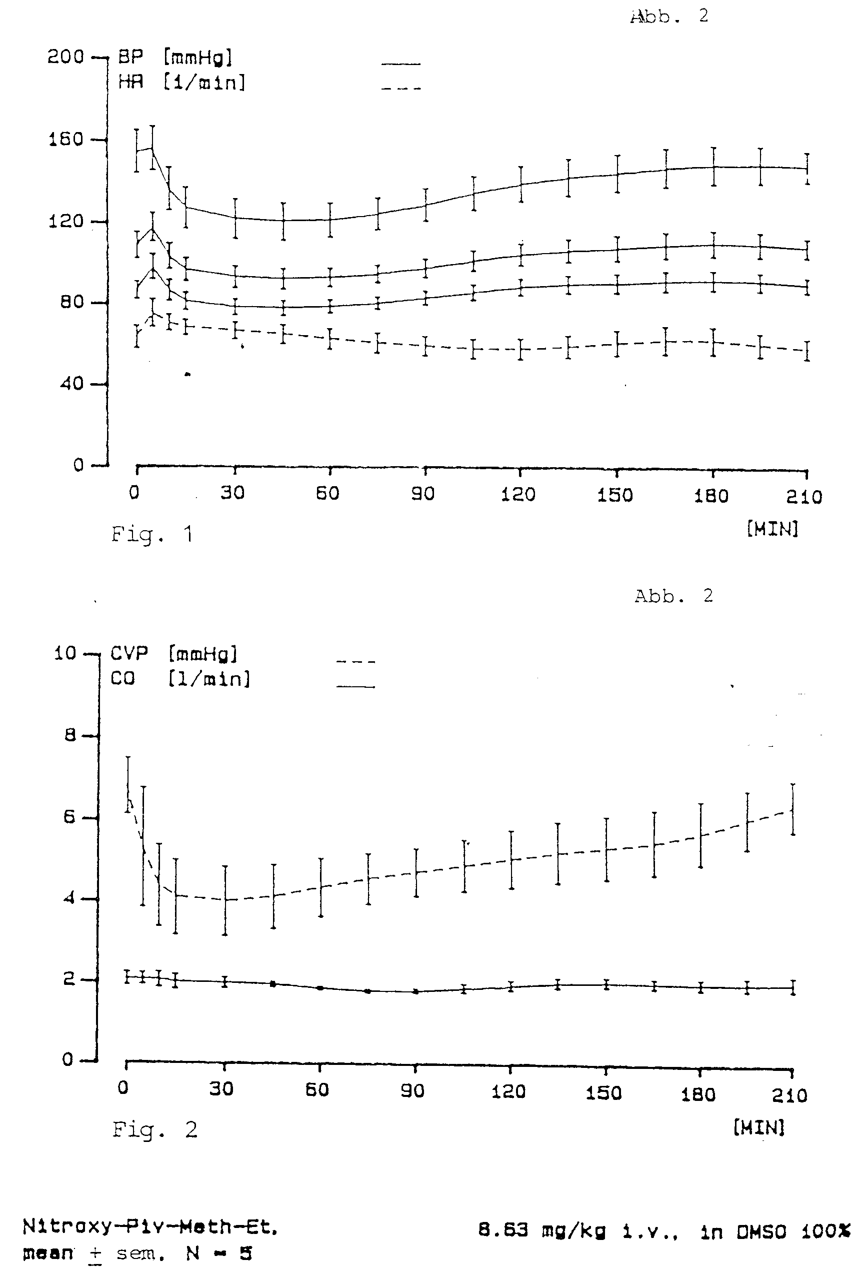

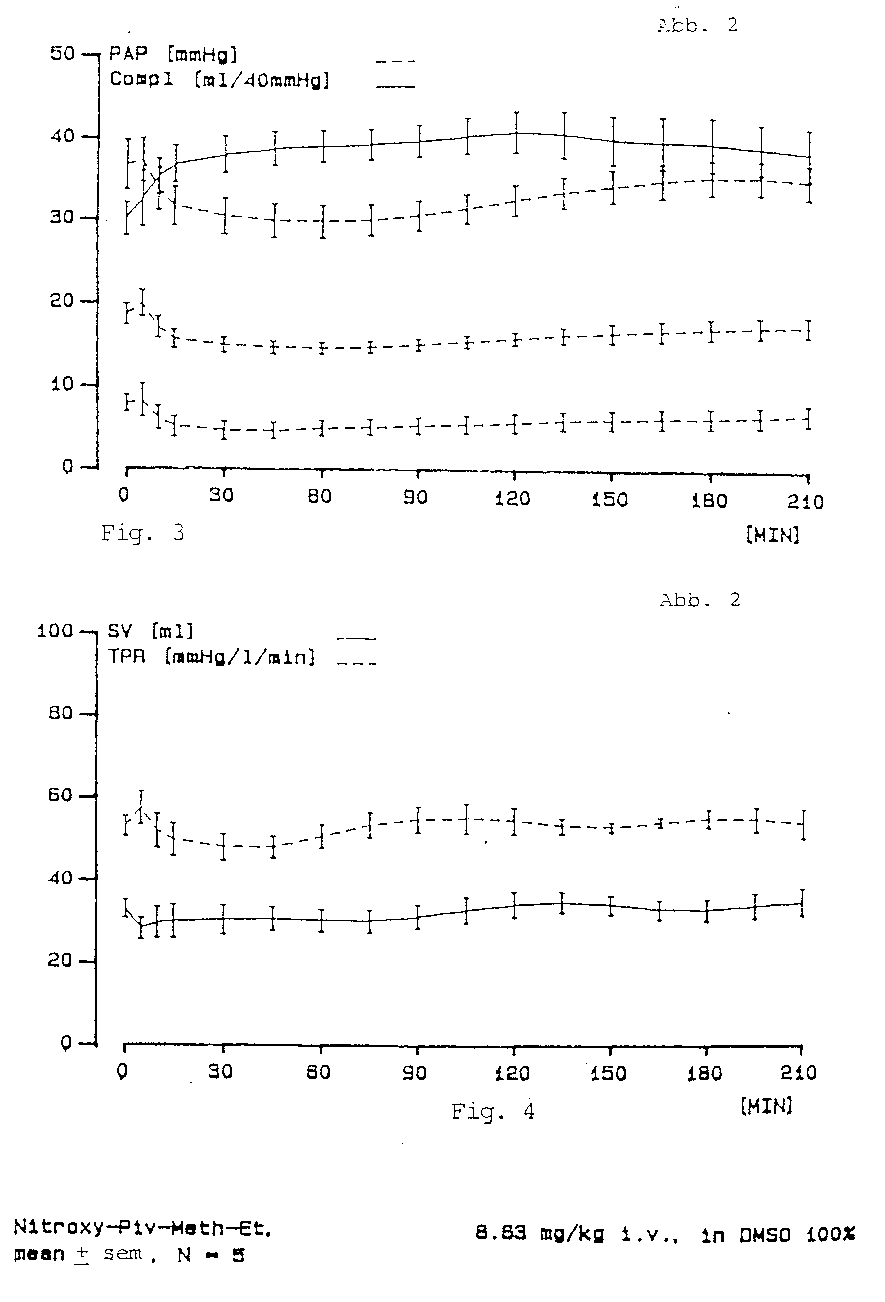

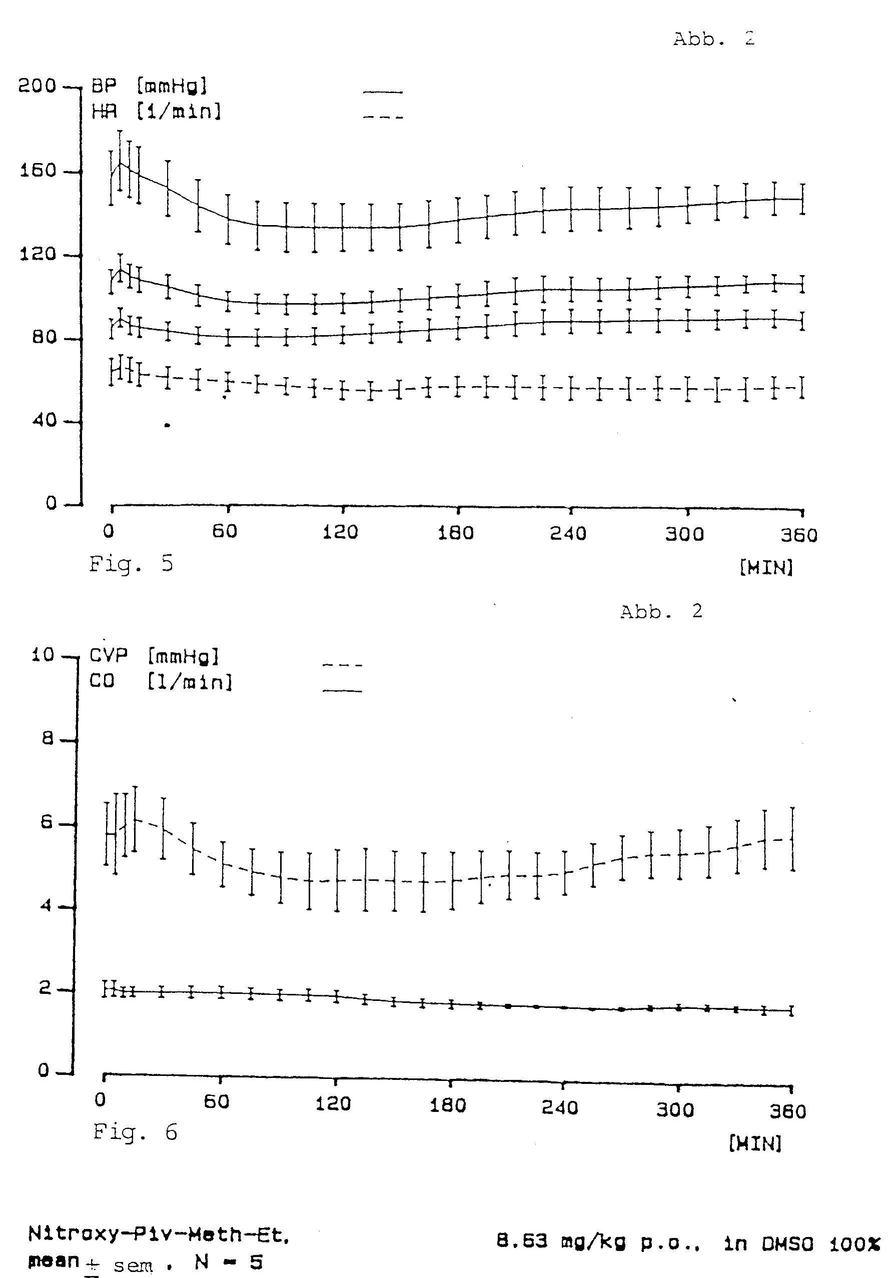

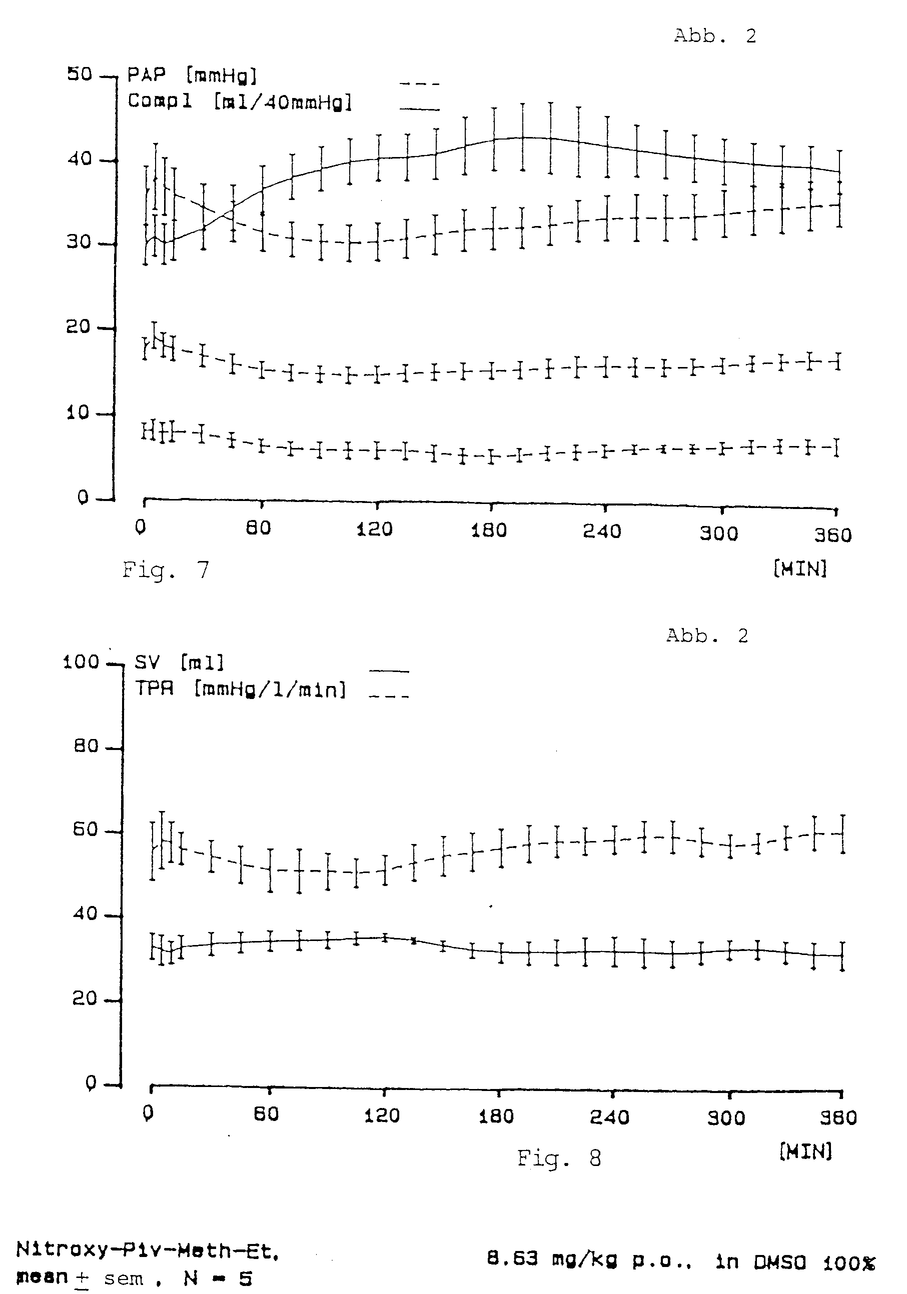

### Pharmakologische Versuchsanordnung 2

Wirkung neuer organischer Nitrate auf den Koronarflußanstieg am isoliert perfundierten Herzen zum Nachweis der ausbleibenden Toleranz.

Ziel der vorliegenden Untersuchung war es, die Wirkung und die Toleranzentstehung neuer organischer Nitratverbindungen am isoliert perfundierten Rattenherzen zu untersuchen. Dazu wurde ein Rattenherz isoliert und als "Working Heart" präpariert.

Das Herz leistet in dieser Versuchsanordnung eine definierte Kreislaufarbeit, woraus ein definierter Sauerstoffverbrauch und Koronarfluß resultiert. Die Wirkung nitratartiger Verbindungen läßt sich in diesem Modell am pharmakoninduzierten Koronarflußanstieg messen.

An einem isolierten, arbeitenden Rattenherzen wurde der Widerstand der Koronargefäße als Parameter für den Nachweis der Nitratwirkung gewählt. Ein Rattenherz mit einem Gewicht von ca. 1 g wird über den linken Vorhof mit einer plasmaähnlichen Lösung, welche Nährstoffe enthält und mit Sauerstoff aufgesättigt ist, perfundiert. Der linke Ventrikel pumpt die Lösung gegen einen definierten Druck in die Aorta. Entsprechend den physiologischen Bedingungen fließt ein Teil dieser Lösung durch die Koronargefäße für die Eigenversorgung des Herzens. Bei definierter Herzarbeit ist dieser Anteil, aus dem sich der koronare Widerstand errechnen läßt, konstant. Der Zusatz eines Nitrates oder anderer koronardilatatorischer Pharmaka bewirkt ein Absinken dieses Koronarwiderstandes. Führt man daher dem Herzen eine konstante Konzentration eines organischen Nitrates zu, so zeigt sich nach einer initialen Abnahme des Widerstandes innerhalb von 20 Minuten ein partieller Wirkungsverlust. Die Substanzen zeigen an diesem Modell ebenfalls einen koronardilatatorischen Effekt, der jedoch nicht von einem Wirkungsverlust gefolgt ist. Auch nach 60 Minuten ist die maximale Senkung des Koronarwiderstandes noch vollständig vorhanden. Die geprüften Substanzen wurden in äquimolarer Dosierung mit 10⁻⁴ M Nitroglycerintrinitrat verglichen. Eine kontinuierliche Infusion von Nitroglycerin bewirkt einen schnellen Koronarflußanstieg um 7.6 ± 1.88 ml/min * ^{g} ww (X̅ ± SD). Innerhalb von 20 Minuten nimmt der Fluß um 55.9 % ab. Bei weiterer Perfusion verläuft die Nitroglycerinwirkung unverändert. Die neuen Nitrate zeigen in diesem Versuchsmodell ebenfalls einen Koronarflußanstieg, der allerdings nur von einem sehr geringfügigen Rückgang der Wirkung gefolgt ist. Dieses Ergebnis weist daraufhin, daß die beschriebenen neuen Verbindungen kein Toleranzverhalten wie herkömmliche Nitrate zeigen.

**Tabelle 1**

| Wirkung neuer organischer Nitrate im Vergleich zu Nitroglycerin auf den Koronarfluß am isoliert perfundierten Rattenherzen. X̅ ± SEM, n = 7. | | |
|---|---|---|
| | Maximaler Koronarflußanstieg (ml/min*g_{ww}) | Wirkungsrückgang (%) |
| 100 »M Nitroglycerin | 7.6 ± 0.71 | 56.0 |
| 100 »M N-(3-Nitratopivaloyl)-Cysteinethylester | 6.6 ± 0.88 | 5.2 |
| 100 »M N-(3-Nitratopivaloyl)-methioninethylester | 8.3 ± 0.92 | 7.0 |
| ± SEM = Standardabweichung des Mittelwertes n = Anzahl der Standardabweichungen | | |

Schließlich wurde die Wirkung von N-(3-Nitratopivaloyl)-cysteinethylester (Nitrato-Piv-Cy-Et) am Meerschweinchenherzen charakterisiert. Nitrato-Piv-Cy-Et führt am Working Heart-Modell (Meerschweinchenherz) zu einer konzentrationsabhängigen Steigerung des Koronarflusses bereits in einem sehr niedrigen Dosierungsbereich. Eine Flußsteigerung um 25 % wird schon mit 380 »g Nitrato-Piv-Cy-Et/l Perfusionsmedium entsprechend 1,3 »Mol/l erreicht. Die entsprechende Konzentration liegt für Glyceroltrinitrat (GTN) mit 5 mg/l um mindestens den Faktor 12 höher. Es handelt sich somit bei Nitrato-Piv-Cy-Et um eine besonders gefäßaktive Verbindung. Eine Abschwächung des koronardilatatorischen Effektes als Ausdruck einer Toleranzentwicklung war unter keiner Dosierung während der einstündigen Verumperfusion festzustellen. Es kann daraus geschlossen werden, daß Nitrato-Piv-Cy-Et im Unterschied zu GTN keine Gefäßtoleranz hervorruft.

Nitrato-Piv-Cy-Et führt an der isolierten Guanylatzyklase zu einer konzentrationsabhängigen Aktivierung des Enzyms entsprechend einer vermehrten Bildung von cGMP pro Zeiteinheit (vergl. Abb.). Das besondere dieser Verbindung ist dabei im Vergleich zu den klassischen organischen Nitroverbindungen, daß die Aktivierung in vitro auch in Abwesenheit von Cystein stattfindet. Das erklärt zugleich die Beobachtung, daß Nitrato-Piv-Cy-Et und chem. Verbindungen z.B. am sogen. "Working Heart Modell" keine Toleranz hervorrufen, ein Befund, der für die klinische Langzeitanwendung von besonders großer praktischer Bedeutung ist. Die für die halbmaximale Aktivierung (ED₅₀) der Guanylatzyklase erforderliche Konzentration liegt um 200 »mol/l. Der vergleichbare Wert für GTN (in Gegenwart von 5 mmol/l Cystein) bewegt sich vergleichsweise um 80 »mol/l.

## Patentansprüche (Patentansprüche für folgende(n) Vertragsstaat(en): AT, BE, CH, DE, FR, GB, IT, LI, LU, NL, SE)

1. Verbindungen der allgemeinen Formel (I) 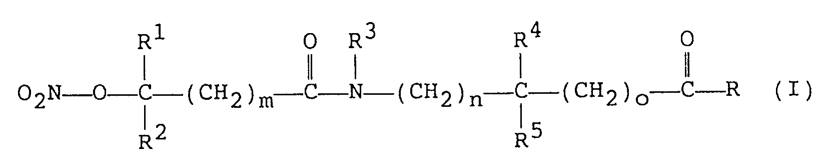 worin
R Hydroxy, C₁-C₃-alkoxy, Amino;
R¹ Wasserstoff, Alkyl mit 1 bis 6 Kohlenstoffatomen;
R² Wasserstoff oder Methyl;
R³ Wasserstoff;
R⁴ Wasserstoff, Phenyl oder Methoxyphenyl;
R⁵ Mercaptomethyl, Mercaptoethyl, Methylthioethyl, dessen S-Acylderivaten, insbesondere S-acetyl, S-propionyl, S-butyryl, S-capronyl, S-capryl, S-isobutyryl, S-pivaloyl, S-benzoyl bedeuten,
R und R⁵ miteinander unter Bildung eines Thiolactons, verbunden sein können,
R und R⁴ miteinander unter Bildung eines Esters oder Amids verbunden sein können,
R³ und R⁴ miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
m, n und o die Zahlen 0 - 10 bedeuten
und die pharmazeutisch annehmbaren Salze davon.

2. Verbindungen nach Anspruch 1, dadurch gekennzeichnet, daß deren Nitratofettsäurebestandteile eine Kettenlänge von C₂ - C₆ haben, geradkettig, verzweigt, racemisch oder optisch isomer sind.

3. Verbindungen nach Ansprüchen 1 und 2, dadurch gekennzeichnet, daß die Aminosäuren bevorzugt Cystein, Methionin oder Homocystein sind.

4. Verbindungen nach Ansprüchen 1-3, dadurch gekennzeichnet, daß die Aminosäuren in der stereochemischen L-Form vorliegen.

5. Verbindungen nach Ansprüchen 1-4, dadurch gekennzeichnet, daß die Aminosäuren Cystein und/oder Methionin als Methyl-, Ethyl- oder Propylester vorliegen.

6. Verbindungen nach Ansprüchen 1-4, dadurch gekennzeichnet, daß Cystein an der SH-Gruppe mit Alkancarbonsäuren der Kettenlänge C₂ bis C₈ verestert ist.

7. Verbindungen nach Ansprüchen 1-5, dadurch gekennzeichnet, daß sie die folgenden chemischen Formeln aufweisen:
N-(2-Nitratoacetyl)-cysteinethylester
N-(2-Nitratoacetyl)-S-acetyl-cysteinethylester
N-(2-Nitratoacetyl)-S-propionyl-cysteinethylester
N-(2-Nitratoacetyl)-S-pivaloyl-cysteinethylester
N-(2-Nitratoacetyl)-methioninmethylester
N-(2-Nitratopropionyl)-cystein
N-(2-Nitratopropionyl)-cysteinethylester
N-(2-Nitratopropionyl)-methioninethylester
N-(2-Nitratobutyryl)-cystein
N-(2-Nitratobutyryl)-cysteinethylester
N-(2-Nitratobutyryl)-S-acetyl-cysteinethylester
N-(2-Nitratobutyryl)-S-butyryl-cysteinethylester
N-(2-Nitratobutyryl)-methioninethylester
N-(2-Nitratoisobutyryl)-cystein
N-(2-Nitratoisobutyryl)-cysteinethylester
N-(2-Nitratoisobutyryl)-S-benzoyl-cysteinethylester
N-(2-Nitratoisobutyryl)-S-acetyl-cysteinethylester
N-(2-Nitratoisobutyryl)-S-pivaloyl-cysteinethylester
N-(2-Nitratoisobutyryl)-methioninethylester
N-(3-Nitratobutyryl)-cystein
N-(3-Nitratobutyryl)-cysteinethylester
N-(3-Nitratobutyryl)-S-acetyl-cysteinethylester
N-(3-Nitratobutyryl)-S-propionyl-cysteinethylester
N-(3-Nitratobutyryl)-methioninethylester
N-(3-Nitratobutyryl)-homocysteinthiolacton
N-(2-Nitratohexanoyl)-cysteinethylester
N-(2-Nitratohexanoyl)-S-propionyl-cysteinethylester
N-(3-Nitratohexanoyl)-cysteinethylester
N-(3-Nitratohexanoyl)-methioninmethylester
N-(12-Nitratolauroyl)-cystein
N-(12-Nitratolauroyl)-cysteinethylester
N-(12-Nitratolauroyl)-S-acetyl-cystein
N-(12-Nitratolauroyl)-S-pivaloyl-cystein

8. Verbindungen, die die folgenden chemischen Bezeichnungen aufweisen:
N-(3-Nitratopivaloyl)-cystein
N-(3-Nitratopivaloyl)-cysteinethylester
N-(3-Nitratopivaloyl)-cysteinethylester-S-carbonat
N-(3-Nitratopivaloyl)-S-acetyl-cysteinethylester
N-(3-Nitratopivaloyl)-S-propionyl-cysteinethylester
N-(3-Nitratopivaloyl)-S-butyryl-cysteinethylester
N-(3-Nitratopivaloyl)-S-isobutyryl-cysteinethylester
N-(3-Nitratopivaloyl)-S-pivaloyl-cysteinethylester
N-(3-Nitratopivaloyl)-S-benzoyl-cysteinethylester
N-(3-Nitratopivaloyl)-methioninethylester
N-(3-Nitratopivaloyl)-methionin
N-(3-Nitratopivaloyl)-homocysteinthiolacton

9. Arzneimittel, gekennzeichnet durch einen Gehalt an einer Verbindung oder einem Gemisch von Verbindungen gemäß Ansprüchen 1 - 8 und/oder deren Derivate und/oder deren pharmazeutisch annehmbaren Salze.

10. Verfahren zur Herstellung von Verbindungen gemäß Ansprüchen 1 - 8, dadurch gekennzeichnet, daß die Verbindungen in an sich bekannter Weise durch Kondensation der entsprechenden Nitratofettsäuren oder deren reaktionsfähigen Derivate mit der Aminogruppe einer Aminosäure bzw. eines Peptids und/oder gegebenenfalls mittels eines weiteren Reaktionsschrittes zur Seitenkettenalkylierung hergestellt werden.

## Patentansprüche (Patentansprüche für folgende(n) Vertragsstaat(en): ES, GR)

1. Verfahren zur Herstellung von Verbindungen sowie deren pharmazeutisch akzeptablen Salze, dadurch gekennzeichnet, daß eine Verbindung entsprechender Nitratofettsäuren der allgemeinen Formel (A) 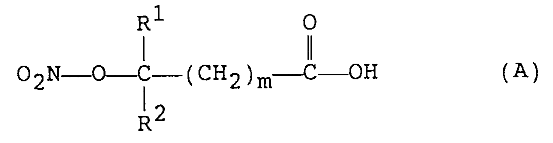 in Form ihrer freien Säure, ihres reaktiven Säurehalogenids, Azids, Esters und/oder Anhydrids mit der Aminogruppe einer Verbindung der allgemeinen Formel (B) 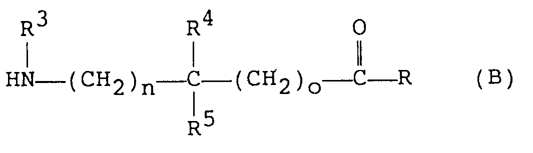 kondensiert wird, um eine Verbindung der allgemeinen Formel (I) zu bilden 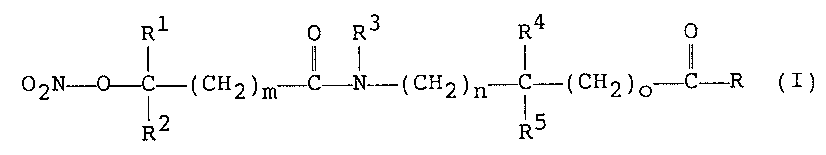 die gegebenenfalls an der Seitenkette mit einer C₁-C₃-Gruppe alkyliert wird, wobei in den allgemeinen Formeln (A), (B) und (I) bedeuten:
R Hydroxy, C₁-C₃-alkoxy, Amino;
R¹ Wasserstoff, Alkyl mit 1 bis 6 Kohlenstoffatomen;
R² Wasserstoff oder Methyl;
R³ Wasserstoff;
R⁴ Wasserstoff, Phenyl oder Methoxyphenyl;
R⁵ Mercaptomethyl, Mercaptoethyl, Methylthioethyl, dessen S-Acylderivaten, insbesondere S-acetyl, S-propionyl, S-butyryl, S-capronyl, S-capryl, S-isobutyryl, S-pivaloyl, S-benzoyl;
R und R⁵ miteinander unter Bildung eines Thiolactons, verbunden sein können,
R und R⁴ miteinander unter Bildung eines Esters oder Amids verbunden sein können,
R³ und R⁴ miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, verbunden sein können,
m, n und o die Zahlen 0 - 10.

2. Verfahren nach Anspruch 1, dadurch gekennzeichnet, daß Nitratofettsäuren mit einer Kettenlänge von C₂-C₆, die linear oder verzweigt, racemisch oder optisch isomer sind, verwendet werden.

3. Verfahren nach Ansprüchen 1 und 2, dadurch gekennzeichnet, daß als Aminosäuren Cystein, Methionin oder Homocystein verwendet werden.

4. Verfahren nach einem der Ansprüche 1-3, dadurch gekennzeichnet, daß als Aminosäuren Säuren mit der stereochemischen L-Form verwendet werden.

5. Verfahren nach einem der Ansprüche 1-4, dadurch gekennzeichnet, daß die Aminosäuren Cystein und/oder Methionin als Methyl-, Ethyl- oder Propylester verwendet werden.

6. Verfahren nach einem der Ansprüche 1-5, dadurch gekennzeichnet, daß Cystein verwendet wird, das an der SH-Gruppe mit Alkancarbonsäuren mit 2-8 C-Atomen verestert ist.

7. Verfahren nach einem der Ansprüche 1-6, dadurch gekennzeichnet, daß die erhaltenen Verbindungen die folgenden chemischen Bezeichnungen tragen:
N-(2-Nitratoacetyl)-cysteinethylester
N-(2-Nitratoacetyl)-S-acetyl-cysteinethylester
N-(2-Nitratoacetyl)-S-propionyl-cysteinethylester
N-(2-Nitratoacetyl)-S-pivaloyl-cysteinethylester
N-(2-Nitratoacetyl)-methioninmethylester
N-(2-Nitratopropionyl)-cystein
N-(2-Nitratopropionyl)-cysteinethylester
N-(2-Nitratopropionyl)-methioninethylester
N-(2-Nitratobutyryl)-cystein
N-(2-Nitratobutyryl)-cysteinethylester
N-(2-Nitratobutyryl)-S-acetyl-cysteinethylester
N-(2-Nitratobutyryl)-S-butyryl-cysteinethylester
N-(2-Nitratobutyryl)-methioninethylester
N-(2-Nitratoisobutyryl)-cystein
N-(2-Nitratoisobutyryl)-cysteinethylester
N-(2-Nitratoisobutyryl)-S-benzoyl-cysteinethylester
N-(2-Nitratoisobutyryl)-S-acetyl-cysteinethylester
N-(2-Nitratoisobutyryl)-S-pivaloyl-cysteinethylester
N-(2-Nitratoisobutyryl)-methioninethylester
N-(3-Nitratobutyryl)-cystein
N-(3-Nitratobutyryl)-cysteinethylester
N-(3-Nitratobutyryl)-S-acetyl-cysteinethylester
N-(3-Nitratobutyryl)-S-propionyl-cysteinethylester
N-(3-Nitratobutyryl)-methioninethylester
N-(3-Nitratobutyryl)-homocysteinthiolacton
N-(2-Nitratohexanoyl)-cysteinethylester
N-(2-Nitratohexanoyl)-S-propionyl-cysteinethylester
N-(3-Nitratohexanoyl)-cysteinethylester
N-(3-Nitratohexanoyl)-methioninmethylester
N-(12-Nitratolauroyl)-cystein
N-(12-Nitratolauroyl)-cysteinethylester
N-(12-Nitratolauroyl)-S-acetyl-cystein
N-(12-Nitratolauroyl)-S-pivaloyl-cystein

8. Verfahren zur Herstellung von Verbindungen, dadurch gekennzeichnet, daß die Verbindungen, die die folgenden chemischen Bezeichnungen aufweisen
N-(3-Nitratopivaloyl)-cystein
N-(3-Nitratopivaloyl)-cysteinethylester
N-(3-Nitratopivaloyl)-cysteinethylester-S-carbonat
N-(3-Nitratopivaloyl)-S-acetyl-cysteinethylester
N-(3-Nitratopivaloyl)-S-propionyl-cysteinethylester
N-(3-Nitratopivaloyl)-S-butyryl-cysteinethylester
N-(3-Nitratopivaloyl)-S-isobutyryl-cysteinethylester
N-(3-Nitratopivaloyl)-S-pivaloyl-cysteinethylester
N-(3-Nitratopivaloyl)-S-benzoyl-cysteinethylester
N-(3-Nitratopivaloyl)-methioninethylester
N-(3-Nitratopivaloyl)-methionin
N-(3-Nitratopivaloyl)-homocysteinthiolacton
in an sich bekannter Weise durch Kondensation der entsprechenden Nitratofettsäuren oder deren reaktionsfähigen Derivate mit der Aminogruppe einer Aminosäure bzw. eines Peptids und/oder gegebenenfalls mittels eines weiteren Reaktionsschrittes zur Seitenkettenalkylierung hergestellt werden.

9. Verfahren zur Herstellung von Arzneimitteln, dadurch gekennzeichnet, daß eine Verbindung oder ein Gemisch von Verbindungen gemäß Ansprüchen 1-8 und/oder deren Derivate und/oder deren pharmazeutisch annehmbaren Salze gegebenenfalls mit pharmazeutischen Hilfsstoffen in eine Arzneiform verbracht werden.

## Claims (Claims for the following Contracting State(s): AT, BE, CH, DE, FR, GB, IT, LI, LU, NL, SE)

1. Compounds of the general formula (I) 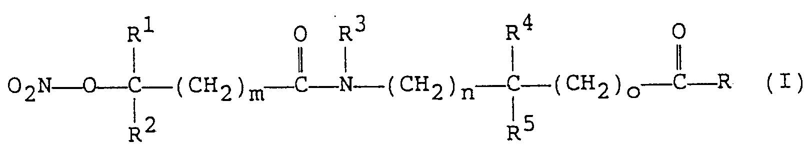 wherein
R represents hydroxy, C₁ - C₃ -alkoxy, amino;
R¹ represents hydrogen, alkyl with 1 to 6 carbon atoms;
R² represents hydrogen or methyl;
R³ represents hydrogen;
R⁴ represents hydrogen, phenyl or methoxyphenyl;
R⁵ represents mercaptomethyl, mercaptoethyl, methylthioethyl its derivatives of S-acyl, particularly S-acetate, S-propionate, S-butyrate, S-caproate, S-caprylate, S-isobutyrate, S-pivalate, S-benzoate,
groups in which R and R⁵ are bonded together in the form of a thiolactone,
groups in which R and R⁴ are bonded together in the form of an ester or an amide,
groups in which R³ and R⁴ are bonded together in the form of an alkylene bridge with 2 to 4 carbon atoms, an alkylene bridge with 2 to 3 carbon atoms and a sulfur atom, an alkylene bridge with 3 to 4 carbon atoms, which contains a double bond or an alkylene bridge with 2 to 3 carbon atoms which can be substituted with a member of the group consisting of hydroxy;
m, n and o are whole numbers from 0 to 10, and pharmaceutically acceptable salts thereof.

2. Compounds according to claim 1 characterized in that the nitrato alkanoic acid components have a chain length of C₂ - C₆, and are selected from the group consisting of straight chain, branched chain, racemic or optical isomers.

3. Compounds according to claims 1 and 2 characterized in that the amino acid is a member of the group consisting of cysteine, methionine or homocysteine.

4. Compounds according to claims 1 to 3 characterized in that the amino acids are in the stereochemical L-form.

5. Compounds according to claims 1 to 4 characterized in that the amino acids selected from the group consisting of cysteine and/or methionine are present as an ester selected from the group consisting of methyl, ethyl and propyl esters.

6. Compounds according to claims 1 to 4 characterized in that cysteine is esterified on the SH - group with an alkanoic acid having a chain length of C₂ - C₈.

7. Compounds according to claims 1 to 5 characterized in that they are selected from the group consisting of:
N-(2-Nitratoacetyl)-cysteinethylester
N-(2-Nitratoacetyl)-S-acetyl-cysteinethylester
N-(2-Nitratoacetyl)-S-propionyl-cysteinethylester
N-(2-Nitratoacetyl)-S-pivaloyl-cysteinethylester
N-(2-Nitratoacetyl)-methioninmethylester
N-(2-Nitratopropionyl)-cysteine
N-(2-Nitratopropionyl)-cysteinethylester
N-(2-Nitratopropionyl)-methioninethylester
N-(2-Nitratobutyryl)-cysteine
N-(2-Nitratobutyryl)-cysteinethylester
N-(2-Nitratobutyryl)-S-acetyl-cysteinethylester
N-(2-Nitratobutyryl)-S-butyryl-cysteinethylester
N-(2-Nitratobutyryl)-methioninethylester
N-(2-Nitratoisobutyryl)-cysteine
N-(2-Nitratoisobutyryl)-cysteinethylester
N-(2-Nitratoisobutyryl)-S-benzoyl-cysteinethylester
N-(2-Nitratoisobutyryl)-S-acetyl-cysteinethylester
N-(2-Nitratoisobutyryl)-S-pivaloyl-cysteinethylester
N-(2-Nitratoisobutyryl)-methioninethylester
N-(3-Nitratobutyryl)-cysteine
N-(3-Nitratobutyryl)-cysteinethylester
N-(3-Nitratobutyryl)-S-acetyl-cysteinethylester
N-(3-Nitratobutyryl)-S-propionyl-cysteinethylester
N-(3-Nitratobutyryl)-methioninethylester
N-(3-Nitratobutyryl)-homocysteinthiolactone
N-(2-Nitratohexanoyl)-cysteinethylester
N-(2-Nitratohexanoyl)-S-propionyl-cysteinethylester
N-(3-Nitratohexanoyl)-cysteinethylester
N-(3-Nitratohexanoyl)-methioninmethylester
N-(12-Nitratolauroyl)-cysteine
N-(12-Nitratolauroyl)-cysteinethylester
N-(12-Nitratolauroyl)-S-acetyl-cysteine
N-(12-Nitratolauroyl)-S-pivaloyl-cysteine

8. Compounds selected from the group consisting of:
N-(3-Nitratopivaloyl)-cysteine
N-(3-Nitratopivaloyl)-cysteinethylester
N-(3-Nitratopivaloyl)-cysteinethylester-S-carbonate
N-(3-Nitratopivaloyl)-S-acetyl-cysteinethylester
N-(3-Nitratopivaloyl)-S-propionyl-cysteinethylester
N-(3-Nitratopivaloyl)-S-butyryl-cysteinethylester
N-(3-Nitratopivaloyl)-S-isobutyryl-cysteinethylester
N-(3-Nitratopivaloyl)-S-pivaloyl-cysteinethylester
N-(3-Nitratopivaloyl)-S-benzoyl-cysteinethylester
N-(3-Nitratopivaloyl)-methioninethylester
N-(3-Nitratopivaloyl)-methionine
N-(3-Nitratopivaloyl)-homocysteinthiolactone

9. Pharmaceutical compositions comprising as an active ingredient a compound or a mixture of compounds and/or derivatives and/or pharmaceutically acceptable salts thereof as defined according claims 1 to 8.

10. Process for the manufacturing of compounds according claims 1 to 8 characterized in that the compounds are produced in a well known manner by condensation of the respective nitrato alcanoic acids or the reactive derivatives thereof with the amino group of an amino acid or with the amino group of a peptide and further, if desired, being side chain alkylated.

## Claims (Claims for the following Contracting State(s): ES, GR)

1. Process for the manufacturing of compounds and pharmaceutically acceptable salts thereof, characterized in that, a compound of respective nitrato alcanoic acids of the general formula (A) 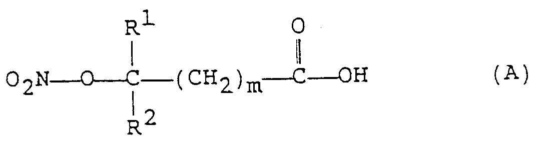 in the form of a member of the group consisting of the free acid, a reactive halide, azide, ester and/or anhydride, is reacting with the amino group of a compound of the general formula (B) 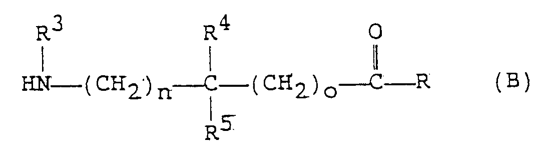 to produce a compound of the general formula (I) 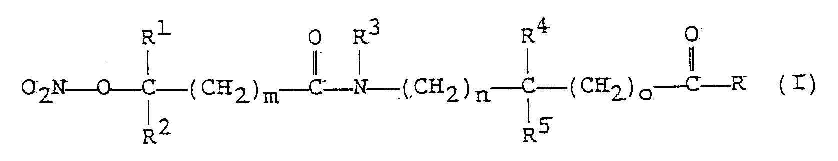 which is alkylated with a C₁ - C₃ - group on the side chain, if desired, and wherein in the general formulas (A), (B) and (I) represent:
R hydroxy, C₁ - C₃ - alkoxy, amino;
R¹ hydrogen, alkyl with 1 to 6 carbon atoms;
R² hydrogen or methyl;
R³ hydrogen;
R⁴ hydrogen, phenyl or methoxyphenyl;
R⁵ mercaptomethyl, mercaptoethyl, methylthioethyl its derivatives of S-acyl, particularly S-acetate, S-propionate, S-butyrate, S-caproate, S-caprylate, S-isobutyrate, S-pivalate, S-benzoate;
groups in which R and R⁵ are bonded together in the form of a thiolactone,
groups in which R and R⁴ are bonded together in the form of an ester or an amide,
groups in which R³ and R⁴ are bonded together in the form of an alkylene bridge with 2 to 4 carbon atoms, an alkylene bridge with 2 to 3 carbon atoms and a sulfur atom, an alkylene bridge with 3 to 4 carbon atoms, which contains a double bond or an alkylene bridge as mentioned above which can be substituted with a member of the group consisting of hydroxy,
m, n and o are whole numbers from 0 to 10.

2. Process for the manufacturing according claim 1, characterized in that are used nitrato alkanoic acids which have a chain length of C₂ - C₆ and are selected from the group consisting of straight-chain, branched chain, racemic or optical isomers.

3. Process for the manufacturing according to claims 1 and 2 characterized in that are used as amino acids cysteine, methionine or homocysteine.

4. Process for the manufacturing according to claims 1 to 3, characterized in that are used amino acids in the stereochemical L-form.

5. Process for the manufacturing according to claims 1 to 4, characterized in that are used amino acids selected from the group consisting of cysteine and/or methionine as an ester selected from the group consisting of methyl, ethyl and propyl esters.

6. Process for the manufacturing according to claims 1 to 5, characterized in that is used cysteine which is esterified on the SH-group with an alkanoic acid having a chain-length of C₂- C₈.

7. Process for the manufacturing according to claims 1 to 6, characterized in that the synthesized compounds are selected from the group consisting of:
N-(2-Nitratoacetyl)-cysteinethylester
N-(2-Nitratoacetyl)-S-acetyl-cysteinethylester
N-(2-Nitratoacetyl)-S-propionyl-cysteinethylester
N-(2-Nitratoacetyl)-S-pivaloyl-cysteinethylester
N-(2-Nitratoacetyl)-methioninmethylester
N-(2-Nitratopropionyl)-cysteine
N-(2-Nitratopropionyl)-cysteinethylester
N-(2-Nitratopropionyl)-methioninethylester
N-(2-Nitratobutyryl)-cysteine
N-(2-Nitratobutymyl)-cysteinethylester
N-(2-Nitratobutyryl)-S-acetyl-cysteinethylester
N-(2-Nitratobutyryl)-S-butyryl-cysteinethylester
N-(2-Nitratobutyryl)-methioninethylester
N-(2-Nitratoisobutyryl)-cysteine
N-(2-Nitratoisobutyryl)-cysteinethylester
N-(2-Nitratoisobutyryl)-S-benzoyl-cysteinethylester
N-(2-Nitratoisobutyryl)-S-acetyl-cysteinethylester
N-(2-Nitratoisobutyryl)-S-pivaloyl-cysteinethylester
N-(2-Nitratoisobutyryl)-methioninethylester
N-(3-Nitratobutyryl)-cysteine
N-(3-Nitratobutyryl)-cysteinethylester
N-(3-Nitratobutyryl)-S-acetyl-cysteinethylester
N-(3-Nitratobutyryl)-S-propionyl-cysteinethylester
N-(3-Nitratobutyryl)-methioninethylester
N-(3-Nitratobutyryl)-homocysteinthiolactone
N-(2-Nitratohexanoyl)-cysteinethylester
N-(2-Nitratohexanoyl)-S-propionyl-cysteinethylester
N-(3-Nitratohexanoyl)-cysteinethylester
N-(3-Nitratohexanoyl)-methioninmethylester
N-(12-Nitratolauroyl)-cysteine
N-(12-Nitratolauroyl)-cysteinethylester
N-(12-Nitratolauroyl)-S-acetyl-cysteine
N-(12-Nitratolauroyl)-S-pivaloyl-cysteine

8. Process for the manufacturing of active compounds, characterized in that the active compounds which are indicated as follows
N-(3-Nitratopivaloyl)-cysteine
N-(3-Nitratopivaloyl)-cysteinethylester
N-(3-Nitratopivaloyl)-cysteinethylester-S-carbonate
N-(3-Nitratopivaloyl)-S-acetyl-cysteinethylester
N-(3-Nitratopivaloyl)-S-propionyl-cysteinethylester
N-(3-Nitratopivaloyl)-S-butyryl-cysteinethylester
N-(3-Nitratopivaloyl)-S-isobutyryl-cysteinethylester
N-(3-Nitratopivaloyl)-S-pivaloyl-cysteinethylester
N-(3-Nitratopivaloyl)-S-benzoyl-cysteinethylester
N-(3-Nitratopivaloyl)-methioninethylester
N-(3-Nitratopivaloyl)-methionine
N-(3-Nitratopivaloyl)-homocysteinthiolactone
are produced in a well known manner by condensation of the respective nitrato alcanoic acids or the reactive derivatives thereof with the amino group of an amino acid or with the amino group of a peptide and further, if desired, being side chain alkylated.

9. Process for the manufacturing of pharmaceutical compositions, characterized in that as an active ingredient a compound or a mixture of compounds according claims 1 to 8 and/or derivatives and/or pharmaceutically acceptable salts thereof comprising pharmaceutical carriers are put in a pharmaceutical form.

## Revendications (Revendications pour l'(les) Etat(s) contractant(s) suivant(s): AT, BE, CH, DE, FR, GB, IT, LI, LU, NL, SE)

1. Composés de la formule générale (I) 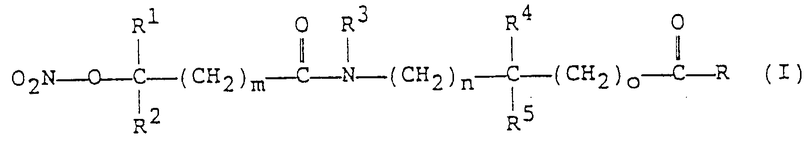 dans laquelle
R représente un radical hydroxyle, alcoxy en C₁-C₃, amino,
R¹ représente un atome d'hydrogène, un radical alkyle qui comporte de 1 à 6 atomes de carbone,
R² représente un atome d'hydrogène ou le radical méthyle,
R³ représente un atome d'hydrogène,
R⁴ représente un atome d'hydrogène, le radical phényle ou méthoxyphényle,
R⁵ représente le radical mercaptométhyle, mercaptoéthyle, méthylthioéthyle, ses dérivés du type S-acyle, plus particulièrement, S-acétyle, S-propionyle, S-butyryle, S-capronyle, S-capryle, S-isobutyryle, S-pivaloyle, S-benzoyle,
R et R⁵ peuvent être mutuellement liés sous formation d'une thiolactone,
R et R⁴ peuvent être mutuellement liés sous formation d'un ester ou d'un amide,
R³ et R⁴ peuvent être mutuellement liés sous formation d'un pont alkylène comportant de 2 à 4 atomes de carbone, d'un pont alkylène comportant 2 ou 3 atomes de carbone et un atome de soufre, d'un pont alkylène comportant 3 ou 4 atomes de carbone, qui, comme plus haut, contient une double liaison ou un pont alkylène, hydroxysubstitué,
m, n et o ont des valeurs numériques qui varient de 0 à 10
et leurs sels pharmaceutiquement acceptables.

2. Composés suivant la revendication 1, caractérisés en ce que leurs composants d'acide nitratogras possèdent une longueur de chaîne de C₂ à C₆ et qu'ils sont à chaîne linéaire, à chaîne ramifiée, racémiques ou des isomères optiques.

3. Composés suivant les revendications 1 et 2, caractérisés en ce que les aminoacides sont, de préférence, la cystéine, la méthionine, ou l'homocystéine.

4. Composés suivant les revendications 1 à 3, caractérisé en ce que les aminoacides se présentent sous la forme stéréochimique L.

5. Composés suivant les revendications 1 à 4, caractérisés en ce que les aminoacides cystéine et/ou méthionine se présentent sous forme des esters méthylique, éthylique ou propylique.

6. Composés suivant l'une quelconque des revendications 1 à 4, caractérisés en ce que la cystéine est estérifiée sur le groupe SH par des acides alcanecarboxyliques d'une longueur de chaîne de C₂ à C₈.

7. Composés suivant les revendications 1 à 5, caractérisés en ce qu'ils possèdent les formules chimiques suivantes :
ester éthylique de la N-(2-nitratoacétyl)-cystéine
ester éthylique de la N-(2-nitratoacétyl)-S-acétyl-cystéine
ester éthylique de la N-(2-nitratoacétyl)-S-propionyl-cystéine
ester éthylique de la N-(2-nitratoacétyl)-S-pivaloyl-cystéine
ester méthylique de la N-(2-nitratoacétyl)-méthionine N-(2-nitratopropionyl)-cystéine
ester éthylique de la N-(2-nitratopropionyl)-cystéine
ester éthylique de la N-(2-nitratopropionyl)-méthionine N-(2-nitratobutyryl)-cystéine
ester éthylique de la N-(2-nitratobutyryl)-cystéine
ester éthylique de la N-(2-nitratobutyrul)-S-acétyl-cystéine
ester éthylique de la N-(2-nitratobutyryl)-S-butyryl-cystéine
ester éthylique de la N-(2-nitratobutyryl)-méthionine N-(2-nitratoisobutyryl)-cystéine
ester éthylique de la N-(2-nitratoisobutyryl)-cystéine
ester éthylique de la N-(2-nitratoisobutyryl)-S-benzoyl-cystéine
ester éthylique de la N-(2-nitratoisobutyryl)-S-acétyl-cystéine
ester éthylique de la N-(2-nitratoisobutyryl)-S-pivaloyl-cystéine
ester éthylique de la N-(2-nitratoisobutyryl)-méthionine N-(3-nitratobutyryl)-cystéine
ester éthylique de la N-(3-nitratobutyryl)-cystéine
ester éthylique de la N-(3-nitratobutyryl)-S-acétyl-cystéine
ester éthylique de la N-(3-nitratobutyryl)-S-propionyl-cystéine
ester éthylique de la N-(3-nitratobutyryl)-méthionine N-(3-nitratobutyryl)-homocystéinethiolactone
ester éthylique de la N-(2-nitratohexanoyl)-cystéine
ester éthylique de N-(2-nitratohexanoyl)-S-propionyl-cystéine
ester éthylique de N-(3-nitratohexanoyl)-cystéine
ester méthylique de la N-(3-nitratohexanoyl)-méthionine N-(12-nitratolauroyl)-cystéine
ester éthylique de la N-(12-nitratolauroyl)-cystéine
N-(12-nitratolauroyl)-S-acétyl-cystéine
N-(12-nitratolauroyl)-S-pivaloyl-cystéine

8. Composés qui répondent aux caractérisations chimiques suivantes :
N-(3-nitratopivaloyl)-cystéine
ester éthylique de la N-(3-nitratopivaloyl)-cystéine S-carbonate de l'ester éthylique de la N-(3-nitratopivaloyl)-cystéine
ester éthylique de la N-(3-nitratopivaloyl)-S-acétyl-cystéine
ester éthylique de la N-(3-nitratopivaloyl)-S-propionyl-cystéine
ester éthylique de la N-(3-nitratopivaloyl)-S-butyryl-
cystéine
ester éthylique de la N-(3-nitratopivaloyl)-S-isobutyryl-cystéine
ester éthylique de la N-(3-nitratopivaloyl)-S-pivaloyl-cystéine
ester éthylique de la N-(3-nitratopivaloyl)-S-benzoyl-cystéine
ester éthylique de la N-(3-nitratopivaloyl)-méthionine
N-(3-nitratopivaloyl)-méthionine
N-(3-nitratopivaloyl)-homocystéinethiolactone

9. Médicaments qui se caractérisent par une teneur en un composé ou un mélange de composés suivant l'une quelconque des revendications 1 à 8 et/ou de leurs dérivés et/ou de leurs sels pharmaceutiquement acceptables.

10. Procédé de préparation des composés suivant les revendications 1 à 8, caractérisé en ce que l'on prépare les composés d'une manière en soi connue par la condensation des acides nitratogras correspondants ou de leurs dérivés réactifs avec le groupe amino d'un aminoacide ou d'un peptide et/ou éventuellement par une étape réactionnelle supplémentaire d'alkylation des chaînes latérales.

## Revendications (Revendications pour l'(les) Etat(s) contractant(s) suivant(s): ES, GR)

1. Procédé de préparation de composés, ainsi que de leurs sels pharmaceutiquement acceptables, caractérisés en ce que l'on condense un composé d'acides nitratogras correspondants de la formule générale (A) 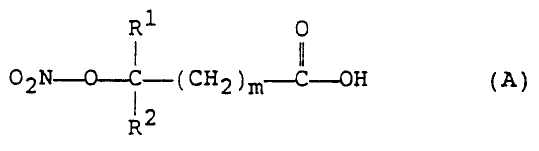 sous forme de leurs acides libres, de leurs anhydrides, esters, azides et/ou halogénures d'acides réactifs, avec le radical amino d'un composé de la formule générale (B) 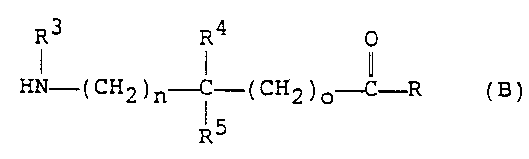 de manière à engendrer un composé de la formule générale (I) 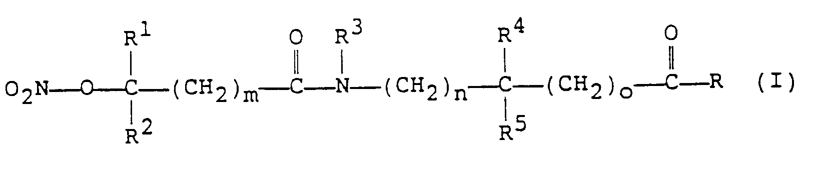 dont on alkyle éventuellement la chaîne latérale avec un groupe en C₁ à C₃, où dans les formules générales (A), (b) et (I) :
R représente un radical hydroxyle, alcoxy en C₁-C₃, amino,
R¹ représente un atome d'hydrogène, un radical alkyle qui comporte de 1 à 6 atomes de carbone,
R² représente un atome d'hydrogène ou le radical méthyle,
R³ représente un atome d'hydrogène,
R⁴ représente un atome d'hydrogène, le radical phényle ou méthoxyphényle,
R⁵ représente le radical mercaptométhyle, mercaptoéthyle, méthylthioéthyle, ses dérivés du type S-acyle, plus particulièrement, S-acétyle, S-propionyle, S-butyryle, S-capronyle, S-capryle, S-isobutyryle, S-pivaloyle, S-benzoyle,
R et R⁵ peuvent être mutuellement liés sous formation d'une thiolactone,
R et R⁴ peuvent être mutuellement liés sous formation d'un ester ou d'un amide,
R³ et R⁴ peuvent être mutuellement liés sous formation d'un pont alkylène comportant de 2 à 4 atomes de carbone, d'un pont alkylène comportant 2 ou 3 atomes de carbone et un atome de soufre, d'un pont alkylène comportant 3 ou 4 atomes de carbone, qui, comme plus haut, contient une double liaison ou un pont alkylène, hydroxysubstitué,
m, n et o ont des valeurs numériques qui varient de 0 à 10.

2. Procédé suivant la revendication 1, caractérisé en ce que l'on utilise des acides nitratogras d'une longueur de chaînes de C₂ à C₆, qui sont linéaires ou ramifiés, qui sont des racémiques ou des isomères optiques.

3. Procédé suivant les revendications 1 et 2, caractérisé en ce que l'on utilise, à titre d'aminoacides, la cystéine, la méthionine ou l'homocystéine.

4. Procédé suivant l'une quelconque des revendications 1 à 3, caractérisé en ce que l'on utilise, à titre d'aminoacides, des acides de la forme stéréochimique L.

5. Procédé suivant l'une quelconque des revendications 1 à 4, caractérisé en ce que l'on utilise les aminoacides cystéine et/ou méthionine sous la forme de leurs esters méthyliques, éthyliques ou propyliques.

6. Procédé suivant l'une quelconque des revendications 1 à 5, caractérisé en ce que l'on utilise la cystéine qui est estérifiée sur le radical SH par des acides alcanecarboxyliques qui comportent de 2 à 8 atomes de carbone.

7. Procédé suivant l'une quelconque des revendications 1 à 6, caractérisé en ce que les composés obtenus répondent aux formes chimiques suivantes :
ester éthylique de la N-(2-nitratoacétyl)-cystéine
ester éthylique de la N-(2-nitratoacétyl)-S-acétyl-cystéine
ester éthylique de la N-(2-nitratoacétyl)-S-propionyl-cystéine
ester éthylique de la N-(2-nitratoacétyl)-S-pivaloyl-cystéine
ester méthylique de la N-(2-nitratoacétyl)-méthionine N-(2-nitratopropionyl)-cystéine
ester éthylique de la N-(2-nitratopropionyl)-cystéine
ester éthylique de la N-(2-nitratopropionyl)-méthionine N-(2-nitratobutyryl)-cystéine
ester éthylique de la N-(2-nitratobutyryl)-cystéine
ester éthylique de la N-(2-nitratobutyrul)-S-acétyl-cystéine
ester éthylique de la N-(2-nitratobutyryl)-S-butyryl-cystéine
ester éthylique de la N-(2-nitratobutyryl)-méthionine N-(2-nitratoisobutyryl)-cystéine
ester éthylique de la N-(2-nitratoisobutyryl)-cystéine
ester éthylique de la N-(2-nitratoisobutyryl)-S-benzoyl-cystéine
ester éthylique de la N-(2-nitratoisobutyryl)-S-acétyl-cystéine
ester éthylique de la N-(2-nitratoisobutyryl)-S-pivaloyl-cystéine
ester éthylique de la N-(2-nitratoisobutyryl)-méthionine N-(3-nitratobutyryl)-cystéine
ester éthylique de la N-(3-nitratobutyryl)-cystéine
ester éthylique de la N-(3-nitratobutyryl)-S-acétyl-cystéine
ester éthylique de la N-(3-nitratobutyryl)-S-propionyl-cystéine
ester éthylique de la N-(3-nitratobutyryl)-méthionine N-(3-nitratobutyryl)-homocystéinethiolactone
ester éthylique de la N-(2-nitratohexanoyl)-cystéine
ester éthylique de N-(2-nitratohexanoyl)-S-propionyl-cystéine
ester éthylique de N-(3-nitratohexanoyl)-cystéine
ester méthylique de la N-(3-nitratohexanoyl)-méthionine N-(12-nitratolauroyl)-cystéine
ester éthylique de la N-(12-nitratolauroyl)-cystéine N-(12-nitratolauroyl)-S-acétyl-cystéine
N-(12-nitratolauroyl)-S-pivaloyl-cystéine

8. Procédé de préparation de composés, caractérisé en ce que les composés qui présentent les formules chimiques suivantes :
N-(3-nitratopivaloyl)-cystéine
ester éthylique de la N-(3-nitratopivaloyl)-cystéine S-carbonate de l'ester éthylique de la N-(3-nitratopivaloyl)-cystéine
ester éthylique de la N-(3-nitratopivaloyl)-S-acétyl-cystéine
ester éthylique de la N-(3-nitratopivaloyl)-S-propionyl-cystéine
ester éthylique de la N-(3-nitratopivaloyl)-S-butyryl-cystéine
ester éthylique de la N-(3-nitratopivaloyl)-S-isobutyryl-cystéine
ester éthylique de la N-(3-nitratopivaloyl)-S-pivaloyl-cystéine
ester éthylique de la N-(3-nitratopivaloyl)-S-benzoyl-cystéine
ester éthylique de la N-(3-nitratopivaloyl)-méthionine N-(3-nitratopivaloyl)-méthionine
N-(3-nitratopivaloyl)-homocystéinethiolactone
sont préparés, de manière en soi connue, par la condensation des acides nitratogras correspondants ou leurs dérivés réactifs avec le radical amino d'un aminoacide ou d'un peptide et/ou éventuellement à l'aide d'une étape réactionnelle supplémentaire d'alkylation des chaînes latérales.

9. Procédé de préparation de médicaments, caractérisé en ce que l'on transforme un composé ou un mélange de composés suivant l'une quelconque des revendications 1 à 8 et/ou leurs dérivés et/ou leurs sels pharmaceutiquement acceptables, en une forme de médicament, éventuellement à l'aide d'adjuvants ou véhicules pharmaceutiques.
